# EUROPEAN PATENT SPECIFICATION

(11) **EP 4 588 527 B1**
(45) Date of publication and mention of the grant of the patent: **11.11.2026**
(21) Application number: 23865028.7
(22) Date of filing: 28.06.2023
(51) Int. Cl.: A45D 33/00, A61K 8/02, A61Q 1/02

(54) **METHOD FOR PRODUCING MULTI-COLOR COSMETIC**
VERFAHREN ZUR HERSTELLUNG EINES MEHRFARBIGEN KOSMETIKUMS
PROCÉDÉ DE PRODUCTION D'UN PRODUIT COSMÉTIQUE MULTICOLORE

(30) Priority: 13.09.2022 JP 2022145433
(43) Date of publication of application: 23.07.2025
(73) Proprietor: Albion Co., Ltd., Tokyo, 104-0031 (JP)
(72) Inventor: KINOSHITA, Masashi, Kumagaya-shi, Saitama 369-0108 (JP); ITO, Masaru, Kumagaya-shi, Saitama 369-0108 (JP); MIYAMOTO, Takafumi, Kumagaya-shi, Saitama 369-0108 (JP); MARUYAMA, Rengo, Kumagaya-shi, Saitama 369-0108 (JP); KAGAYA, Kei, Kumagaya-shi, Saitama 369-0108 (JP)
(74) Representative: Grünecker Patent- und Rechtsanwälte PartG mbB
(86) International application number: PCT/JP2023/023964
(87) International publication number: WO 2024/057666

(56) References cited:
- EP-A1- 4 442 239
- WO-A1-2023/100685
- JP-A- 2007 063 170
- JP-A- 2013 079 202
- JP-A- 2013 079 202
- JP-A- H02 172 903
- JP-A- S62 145 008
- JP-A- S63 188 616
- US-A- 5 858 419

## Description

### TECHNICAL FIELD

The present invention relates to a method for producing a multi-color cosmetic.

### BACKGROUND ART

As an example of a conventional method for producing a multi-color cosmetic, a method described in, for instance, the following Patent Document 1 has been known.

To put it briefly, in this producing method of the multi-color cosmetic, two or more powder-attached rod-shaped cosmetics formed by attaching a powdered cosmetic to an outer peripheral side of each rod-shaped cosmetic are accommodated in a square tubular container, and these powder-attached rod-shaped cosmetics are extruded from the container by a predetermined amount and cut in a direction orthogonal to a major axis of the rod-shaped cosmetic, then a multi-color cosmetic bulk is formed.
Another example of a method for producing a multicolor solid powder cosmetic is described in JP 2013/079202 A. The method includes a step of extruding a multicolor cosmetic bulk including a stick-like molding obtained by molding a powder cosmetic in a stick-like shape and a powder cosmetic, contained in a container which is capable of excluding its content, in a specific amount of the content in the longitudinal direction of the stick-like molding from the container, and cutting the excluded material in a direction orthogonal to the longitudinal direction of the stick-like molding.
Production of a pressed cosmetic is described in document JPS 62145008A.
Document JP 2007/063170A describes a further example of an apparatus and method for producing powder cosmetics.
A machine for the formation of tablets of a cosmetic product is also described in document US 5858419 A.

### CITATION LIST

### PATENT DOCUMENT

Patent Document 1: Japanese Patent No. 5891469

### SUMMARY OF THE INVENTION

### TECHNICAL PROBLEM

However, the conventional producing method of the multi-color cosmetic does not take into consideration at all that in order to obtain the powder-attached rod-shaped cosmetic, attachment (or adhesion) work of the powdered cosmetic cosmetic, such as rotating (rolling) the rod-shaped cosmetic on the powdered cosmetic filling the container, is automated. Therefore, there is room for improvement in that regard.

The present invention was made in view of the above technical problem of the conventional producing method of the multi-color cosmetic. An object of the present invention is therefore to provide a method of producing the multi-color cosmetic which is capable of automating the attachment (or adhesion) work of the powdered cosmetic.

### SOLUTION TO PROBLEM

The above-mentioned object is solved by the present invention as claimed in the appended independent claims. Embodiments of the present invention are defined by the appended dependent claims.

As one aspect of the present invention, a method of producing a multi-color cosmetic comprises: a powder attaching step of attaching a powdered cosmetic to each outer peripheral side of rod-shaped cosmetics forming multiple colors, wherein the rod-shaped cosmetics are each formed by shaping respective cosmetics forming multiple colors of two colors or more into a rod-shape; and a press molding step of compressing and compacting an assembly of a plurality of parallel-arranged unit cosmetics forming multiple colors, wherein the unit cosmetics are obtained by cutting each of powder-attached rod-shaped cosmetics forming multiple colors, which are formed by attaching the powdered cosmetic to the outer peripheral sides of the rod-shaped cosmetics forming multiple colors, in a direction orthogonal to a longitudinal direction of the powder-attached rod-shaped cosmetic, wherein the powder attaching step includes: a powdered cosmetic filling step at which the powdered cosmetic is laid on a sheet; a rod-shaped cosmetic arranging step at which the rod-shaped cosmetic is laid and arranged on the powdered cosmetic laid on the sheet; and a rod-shaped cosmetic rolling step at which by grasping one end portion, in a direction orthogonal to a longitudinal direction of the rod-shaped cosmetic, of the sheet and folding back the one end portion of the sheet to the other end side, the rod-shaped cosmetic is made to roll on the powdered cosmetic, and the powder-attached rod-shaped cosmetic is formed.

As described above, according to the present invention, at the second step, after laying and arranging the rod-shaped cosmetic on the powdered cosmetic laid on the sheet, by folding back the one end portion of the sheet to the other end side and rolling the rod-shaped cosmetic on the powdered cosmetic, the attachment work of the powdered cosmetic to the rod-shaped cosmetic can be automated.

Further, in the present invention, by folding back the sheet, the rod-shaped cosmetic rolls on the powdered cosmetic, then the powder-attached rod-shaped cosmetic is obtained. Therefore, it is possible to relatively easily control the number of times of rolling of the rod-shaped cosmetic, and this has an advantage of being able to easily control an amount of the powdered cosmetic attached to the rod-shaped cosmetic.

In addition, in the present invention, since the rod-shaped cosmetic is made to roll on the powdered cosmetic and the powder-attached rod-shaped cosmetic is obtained, a shape of the rod-shaped cosmetic could be a square columnar shape other than a cylindrical columnar shape, then a degree of freedom of the shape of the rod-shaped cosmetic can be increased.

Further, from another viewpoint, as another aspect of the present invention, a method of producing a multi-color cosmetic comprises: a powder attaching step of attaching a powdered cosmetic to each outer peripheral side of rod-shaped cosmetics forming multiple colors, wherein the rod-shaped cosmetics are each formed by shaping respective cosmetics forming multiple colors of two colors or more into a rod-shape; and a press molding step of compressing and compacting an assembly of a plurality of parallel-arranged unit cosmetics forming multiple colors, wherein the unit cosmetics are obtained by cutting each of powder-attached rod-shaped cosmetics forming multiple colors, which are formed by attaching the powdered cosmetic to the outer peripheral sides of the rod-shaped cosmetics forming multiple colors, in a direction orthogonal to a longitudinal direction of the powder-attached rod-shaped cosmetic, wherein at the powder attaching step, on an upstream side of a conveyor formed by a strip-shaped sheet rotating along a longitudinal direction of the conveyor, the powdered cosmetic is laid on the conveyor, the rod-shaped cosmetic is arranged along a travelling direction of the conveyor on the powdered cosmetic laid on the conveyor, by further sprinkling the powdered cosmetic onto the rod-shaped cosmetic from above, the rod-shaped cosmetic is buried in the powdered cosmetic, and on a downstream side of the conveyor, by rolling up both side portions in a width direction of the conveyor so that the both side portions of the conveyor overlap each other, the powder-attached rod-shaped cosmetic is formed.

As described above, according to the present invention, at the powder attaching step, the rod-shaped cosmetic is arranged along the travelling direction of the conveyor on the sheet on which the powdered cosmetic is laid, and by further sprinkling the powdered cosmetic onto the rod-shaped cosmetic from above, the rod-shaped cosmetic is buried in the powdered cosmetic, and after that, on the downstream side of the conveyor, by rolling up both side portions in the width direction of the conveyor so that the both side portions of the conveyor overlap each other, the attachment work of the powdered cosmetic to the rod-shaped cosmetic can be automated.

Further, in the present invention, the rod-shaped cosmetic is arranged along the travelling direction of the conveyor. Therefore, the powdered cosmetic can be attached to a relatively long rod-shaped cosmetic. As a result, a relatively long powder-attached rod-shaped cosmetic can be obtained. It is thus possible to cut the relatively long powder-attached rod-shaped cosmetic into a required unit length, thereby improving working efficiency in production.

Further, from another viewpoint, as another aspect of the present invention, a method of producing a multi-color cosmetic comprises: a powder attaching step of attaching a powdered cosmetic to each outer peripheral side of rod-shaped cosmetics forming multiple colors, wherein the rod-shaped cosmetics are each formed by shaping respective cosmetics forming multiple colors of two colors or more into a rod-shape; and a press molding step of compressing and compacting an assembly of a plurality of parallel-arranged unit cosmetics forming multiple colors, wherein the unit cosmetics are obtained by cutting each of powder-attached rod-shaped cosmetics forming multiple colors, which are formed by attaching the powdered cosmetic to the outer peripheral sides of the rod-shaped cosmetics forming multiple colors, in a direction orthogonal to a longitudinal direction of the powder-attached rod-shaped cosmetic, wherein at the powder attaching step, on an upstream side of a conveyor formed by a strip-shaped sheet rotating along a longitudinal direction of the conveyor, the powdered cosmetic is laid on the conveyor, the rod-shaped cosmetic is arranged along a travelling direction of the conveyor on the powdered cosmetic laid on the conveyor, by further sprinkling the powdered cosmetic onto the rod-shaped cosmetic from above, the rod-shaped cosmetic is buried in the powdered cosmetic, and on a downstream side of the conveyor, by making the rod-shaped cosmetic buried in the powdered cosmetic pass through an arc-shaped or a circular gap between a pair of rollers that are arranged as a pair so as to face each other in a width direction of the conveyor and that form the arc-shaped or circular gap between the pair of rollers, the powder-attached rod-shaped cosmetic is formed.

As described above, according to the present invention, the rod-shaped cosmetic is arranged along the travelling direction of the conveyor on the sheet on which the powdered cosmetic is laid, and by further sprinkling the powdered cosmetic onto the rod-shaped cosmetic from above, the rod-shaped cosmetic is buried in the powdered cosmetic, and after that, on the downstream side of the conveyor, by making the rod-shaped cosmetic buried in the powdered cosmetic pass through the gap between the pair of rollers, the attachment work of the powdered cosmetic to the rod-shaped cosmetic can be automated.

Further, in the present invention, only by making the rod-shaped cosmetic buried in the powdered cosmetic pass through the gap of the rollers, automation of the attachment work of the powdered cosmetic to the rod-shaped cosmetic can be relatively easily realized.

Further, in the present invention, the rod-shaped cosmetic is arranged along the travelling direction of the conveyor. Therefore, the powdered cosmetic can be attached to a relatively long rod-shaped cosmetic. As a result, a relatively long powder-attached rod-shaped cosmetic can be obtained. It is thus possible to cut the relatively long powder-attached rod-shaped cosmetic into a required unit length, thereby improving working efficiency in production.

As still another aspect of the method of producing the multi-color cosmetic according to the present invention, the pair of rollers are configured so that concave arc surfaces that are concave outwards in the width direction of the conveyor are arranged so as to face each other in the width direction of the conveyor, and so that the arc-shaped gap is formed between the pair of rollers, also a semicircular gap is formed between the pair of rollers and the conveyor.

As described above, according to the present invention, in order to obtain the powder-attached rod-shaped cosmetic, it is only necessary to make the rod-shaped cosmetic buried in the powdered cosmetic pass through the gap between the pair of rollers arranged so as to face the conveyor, which does not require floating the rod-shaped cosmetic from the conveyor. With this, the attachment work of the powdered cosmetic to the rod-shaped cosmetic can be relatively easily realized.

As still another aspect of the method of producing the multi-color cosmetic according to the present invention, the pair of rollers are configured so that semicircular concave arc surfaces that are concave outwards in the width direction of the conveyor are arranged so as to face each other in the width direction of the conveyor, and so that the circular gap is formed between the pair of rollers.

As described above, in the present invention, in order to obtain the powder-attached rod-shaped cosmetic, by making the rod-shaped cosmetic buried in the powdered cosmetic pass through the circular gap formed by the pair of rollers arranged in the width direction of the conveyor, the powder-attached rod-shaped cosmetic having the circular cross section can be directly obtained. Therefore, the powder-attached rod-shaped cosmetic having the circular cross section can be efficiently formed.

### EFFECTS OF INVENTION

According to the present invention, the attachment work of the powdered cosmetic to the rod-shaped cosmetic can be automated. With this, productivity and quality of the multi-color cosmetic can be improved.

### BRIEF DESCRIPTION OF THE DRAWINGS

[Fig. 1] Fig. 1 is a plan view of a multi-color cosmetic according to the present invention.
[Fig. 2] Figs. 2(a) and 2(b) are schematic diagrams illustrating a first step and a second step of a producing method of the multi-color cosmetic not according to the present invention. Fig. 2(a) is a side view, and Fig. 2(b) is a plan view.
[Fig. 3] Fig. 3 is a schematic diagram illustrating a third step of the producing method of the multi-color cosmetic not according to the present invention.
[Fig. 4] Figs. 4(a) and 4(b) are schematic diagrams illustrating a fourth step of the producing method of the multi-color cosmetic according to a first embodiment of the present invention. Fig. 4(a) is a plan view, and Fig. 4(b) is a sectional view taken along an A-A line of Fig. 4(a).
[Fig. 5] Figs. 5(a) and 5(b) are schematic diagrams illustrating a fifth step and subsequent steps of the producing method of the multi-color cosmetic according to the first embodiment of the present invention. Fig. 5(a) is a plan view, and Fig. 5(b) is a sectional view taken along a B-B line of Fig. 5(a).
[Fig. 6] Fig. 6 is a modified example of the producing method of the multi-color cosmetic according to the first embodiment of the present invention, and shows another example of the fourth step of the producing method.
[Fig. 7] Figs. 7(a) and 7(b) are schematic diagrams illustrating a fifth step and subsequent steps of the producing method of the multi-color cosmetic according to the modified example of the first embodiment of the present invention. Fig. 7(a) is a plan view, and Fig. 7(b) is a sectional view taken along a C-C line of Fig. 7(a).
[Fig. 8] Fig. 8 is a second embodiment of the producing method of the multi-color cosmetic according to the present invention, and illustrates a plan view of a powder attaching device showing another example of the second step.
[Fig. 9] Figs. 9(a) to 9(f) are sectional views taken along a D-D line of Fig. 8, and schematically illustrate anattachment (oradhesion) operation of powdered cosmetic to a rod-shaped cosmetic.
[Fig. 10] Figs. 10(a) and 10(b) are a third embodiment of the producing method of the multi-color cosmetic according to the present invention. Fig. 10(a) is a side view of the second step, and Fig. 10(b) is a plan view of the second step.
[Fig. 11] Fig. 11(a) is a sectional view taken along an E-E line of Fig. 10(a). Fig. 11(b) is a sectional view taken along an F-F line of Fig. 10(a). Fig. 11(c) is a sectional view taken along a G-G line of Fig. 10(a). Fig. 11(d) is a sectional view taken along an H-H line of Fig. 10(a).
[Fig. 12] Figs. 12(a) and 12(b) are a fourth embodiment of the producing method of the multi-color cosmetic according to the present invention. Fig. 12(a) is a side view of the second step, and Fig. 12(b) is a plan view of the second step.
[Fig. 13] Fig. 13 is a sectional view taken along an I-I line of Fig. 12(a).
[Fig. 14] Figs. 14(a) and 14(b) are a modified example of the producing method of the multi-color cosmetic according to the fourth embodiment of the present invention. Fig. 14(a) is a plan view of the second step, and Fig. 14(b) is a sectional view taken along a J-J line of Fig. 14(a).

### EMBODIMENTS FOR CARRYING OUT THE INVENTION

Embodiments of a method of producing a multi-color cosmetic according to the present invention will be described below with reference to the drawings.

### (Description of Multi-color cosmetic)

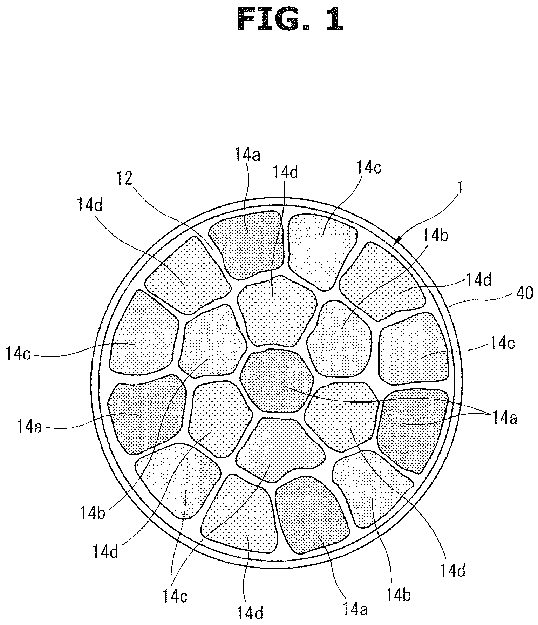

Fig. 1 illustrates a multi-color cosmetic produced by a producing method of a multi-color cosmetic according to the present invention.

For instance, as illustrated in Fig. 1, a multi-color cosmetic 1 produced by the present invention is a multi-color cosmetic in which a plurality of unit cosmetics 14a, 14b, 14c and 14d (four colors in the present embodiment) each having arbitrary color are arranged at predetermined positions, and these unit cosmetics 14a, 14b, 14c and 14d are integral with each other then formed as a single solid cosmetic. In this multi-color cosmetic 1, the unit cosmetics 14a, 14b, 14c and 14d are partitioned by boundaries of a powdered cosmetic 12 having a predetermined color (which is different from colors of the unit cosmetics 14a, 14b, 14c and 14d). The multi-color cosmetic 1 is housed in, for instance, a metal housing tray 40, and further housed in a case (not shown) such as a compact case. Material of these housing tray 40 and case (not shown) can be arbitrarily selected according to specifications etc. of a product.

### [First Embodiment]

### (Description of Method of Producing Multi-color cosmetic)

A first embodiment of the method of producing the multi-color cosmetic 1 not according to the present invention will be described in detail below. As mentioned above, although the multi-color cosmetic 1 is the multi-color cosmetic in which the plurality of unit cosmetics 14a, 14b, 14c and 14d (four colors in the present embodiment) each having arbitrary color are integral with each other then formed as a single solid cosmetic, a production process of each of the unit cosmetics 14a, 14b, 14c and 14d is the same. Therefore, in the following description, for the sake of convenience, the production process of only one unit cosmetic 14 will be described, and individual description of the production process of each of the unit cosmetics 14a, 14b, 14c and 14d is omitted.

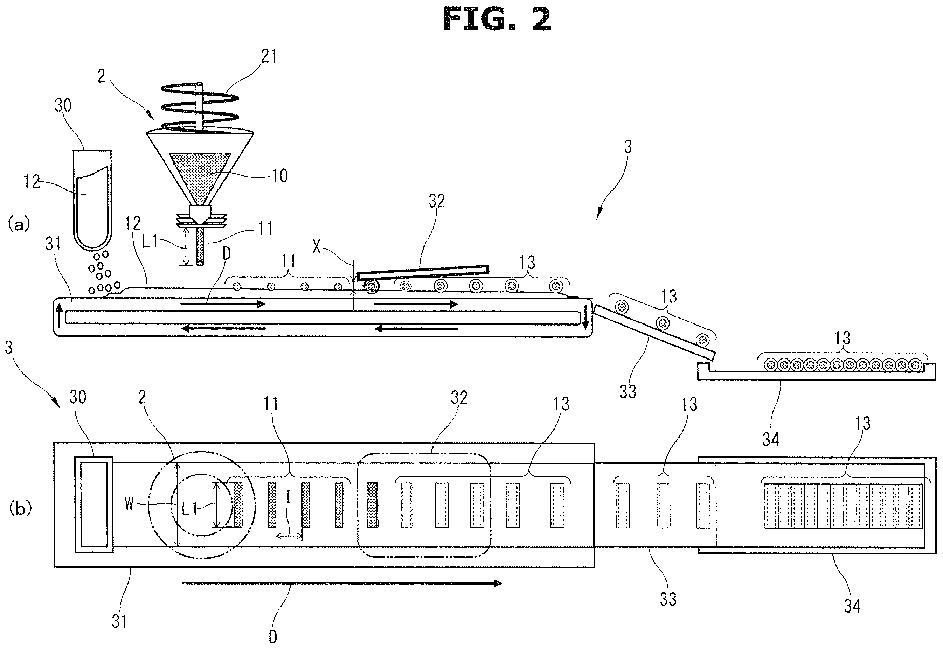

Figs. 2(a) and 2(b) are schematic diagrams illustrating a first step and a second step of the producing method of the multi-color cosmetic according to the present invention. Fig. 2(a) is a side view, and Fig. 2(b) is a plan view.

As illustrated in Figs. 2(a) and 2(b), at the first step (a rod-shaped cosmetic molding step) of the producing method of the multi-color cosmetic, a rod-shaped cosmetic 11 is molded by forming the powdered cosmetic having a predetermined color into a rod-shape. As this molding method of the rod-shaped cosmetic 11, arbitrary methods such as press molding and extrusion molding can be adopted according to the produced multi-color cosmetic, i.e. specifications of a product. The present embodiment exemplifies an example in which the rod-shaped cosmetic 11 is formed by the extrusion molding.

That is, at the first step, the substantially cylindrical columnar rod-shaped cosmetic 11 is molded by an extrusion mold machine 2. The extrusion mold machine 2 has an upright shape disposed along a vertical direction. A powdered cosmetic 10 kneaded and mixed by a screw 21 in the extrusion mold machine 2 is discharged (extruded) from a lower end portion of the extrusion mold machine 2, and this discharged powdered cosmetic 10 is cut at a predetermined length L1, then the substantially cylindrical columnar rod-shaped cosmetic 11 is formed. Here, the extrusion mold machine 2 is disposed above a conveyor 31, which is related to the after-described second step, so as to face the conveyor 31 in the vertical direction. The rod-shaped cosmetic 11 formed at the first step can be then directly placed on the conveyor 31 related to the second step, thereby realizing continuous shift from the first step to the second step.

It is noted that it ispreferable that the extrusion mold machine 2 be provided by the number of colors of the rod-shaped cosmetic 11 formed at the first step. That is, by disposing the extrusion mold machines 2 for respective colors forming the multi-color cosmetic 1, it does not become necessary to change the color in one extrusion mold machine 2. It is therefore possible to increase productivity of the multi-color cosmetic 1.

Further, in view of carrying (conveying), rolling and cutting operations that are described later, it is desirable that the rod-shaped cosmetic 11 have such hardness having a certain shape that its shape can be maintained to some extent. Also, the rod-shaped cosmetic 11 could be adjusted by adding an appropriate amount of a volatile solvent as necessary. In addition, as long as the rod-shaped cosmetic 11 has a desired cross-sectional shape according to pattern or design of the cosmetic, a shape of the rod-shaped cosmetic 11 could be "a cylindrical columnar shape" exemplified in the present embodiment or "a square columnar shape". It is noted that it is preferable that "the predetermined length L1" be a length which is smaller than a width size W of the after-described conveyor 31 and by which the after-described plurality of unit cosmetics 14 can be obtained without wasting the unit cosmetic 14 (with high yield) .

Subsequently, at the second step (a powder attaching step) of the producing method of the multi-color cosmetic, the powdered cosmetic 12 is attached to an outer peripheral side of the rod-shaped cosmetic 11 by a powder attaching device 3 shown in Figs. 2(a) and 2(b). That is, the powder attaching device 3 according to the present embodiment has the conveyor 31 rotating in a direction of an arrow D indicated in Figs. 2(a) and 2(b) along a longitudinal direction of the conveyor 31, a powder container 30 provided at an end portion on an upstream side of the conveyor 31 and storing therein the powdered cosmetic 12, and a pressing jig 32 provided on a downstream side of the conveyor 31 and rolling the rod-shaped cosmetic 11 on the conveyor 31 by pressing the rod-shaped cosmetic 11 carried by the conveyor 31.

That is, at the second step, the rod-shaped cosmetics 11 formed at the first step are arranged parallel to each other at a predetermined interval I in a travelling direction (see the arrow D in Figs. 2(a) and 2(b)) of the conveyor 31 on the conveyor 31 on which the powdered cosmetic 12 is laid, and each rod-shaped cosmetic 11 is made to roll on the powdered cosmetic 12 laid on the conveyor 31, then the powdered cosmetic 12 is attached to the outer peripheral side of the rod-shaped cosmetic 11. Here, the powdered cosmetic 12 is stored in the powder container 30 disposed at the end portion on an opposite side to the travelling direction of the conveyor 31, and is supplied onto the conveyor 31 from a lower portion of the powder container 30. It is noted that although the present embodiment exemplifies, as an example of a way of attaching the powdered cosmetic 12 to the outer peripheral side of the rod-shaped cosmetic 11, an example in which the powdered cosmetic 12 is attached to an entire outer peripheral side of the rod-shaped cosmetic 11, the powdered cosmetic 12 could be attached not only to the entire outer peripheral side of the rod-shaped cosmetic 11, but also to a part of the outer peripheral side of the rod-shaped cosmetic 11.

More specifically, at the second step, the pressing jig 32 is provided at some midpoint of the conveyor 31 so as to face an upper side of the conveyor 31 at a space X at which the rod-shaped cosmetic 11 carried by the conveyor 31 can be in contact with the pressing jig 32. The pressing jig 32 is set to an inclined state so that the space X between the pressing jig 32 and the conveyor 31 gradually increases toward the travelling direction of the conveyor 31. With this setting, the pressing jig 32 comes into contact with the rod-shaped cosmetic 11 at an end portion of the pressing jig 32 on an opposite side to the travelling direction of the conveyor 31, whereas the pressing jig 32 does not come into contact with the rod-shaped cosmetic 11 (a powder-attached rod-shaped cosmetic 13) at an end portion of the pressing jig 32 in the travelling direction of the conveyor 31.

Further, at this second step, when the rod-shaped cosmetic 11 passes under the pressing jig 32 during conveyance of the rod-shaped cosmetic 11 by the conveyor 31, a pressing force acts on the rod-shaped cosmetic 11 from above by the pressing jig 32, and also a rotation force (torque) in a tangential direction of a cross section of the rod-shaped cosmetic 11 (i.e. in the travelling direction of the conveyor 31) is generated at and acts on a lower side of the rod-shaped cosmetic 11 by and according to movement of the conveyor 31, then the rod-shaped cosmetic 11 rolls on the powdered cosmetic 12 laid on the conveyor 31. By this rolling, the powdered cosmetic 12 is attached to the outer peripheral side of the rod-shaped cosmetic 11, and the powder-attached rod-shaped cosmetic 13 is formed. The powder-attached rod-shaped cosmetics 13 formed are carried (conveyed) by the conveyor 31 with the powder-attached rod-shaped cosmetics 13 arranged at the same interval as the above-mentioned interval I, roll on an inclined plate 33 provided adjacently to a downstream end portion of the conveyor 31 in a downward inclined state, and are sequentially discharged to a discharge tray 34.

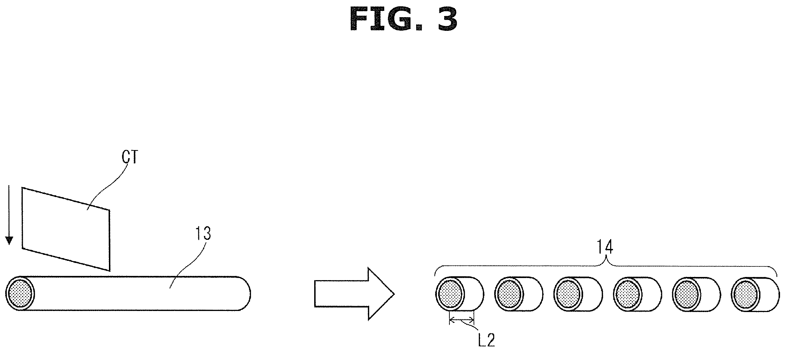

Fig. 3 is a schematic diagram illustrating a third step of the producing method of the multi-color cosmetic according to the present embodiment, and shows a perspective view of the powder-attached rod-shaped cosmetic 13 and powder-attached unit cosmetics 14.

As illustrated in Fig. 3, at the third step (a cosmetic cutting step) of the producing method of the multi-color cosmetic, the powder-attached rod-shaped cosmetic 13 discharged to the discharge tray 34 at the second step is cut into pieces of a predetermined length L2 by a cutter CT, and the powder-attached unit cosmetics 14 are formed. It is noted that "the predetermined length L2" is set to a size that is higher (longer) than a height of a peripheral wall 402 of the housing tray 40 and that becomes the same size (the same height) as that of the peripheral wall 402 of the housing tray40 after undergoing a press molding operation and a surface cutting operation which are described later.

Further, the present embodiment exemplifies an example in which at the third step, the powder-attached rod-shaped cosmetic 13 is cut along a direction orthogonal to a major axis of the powder-attached rod-shaped cosmetic 13. However, the present invention is not limited to this example, but the powder-attached rod-shaped cosmetic 13 could be cut in an arbitrary direction according to pattern or design of a produced solid cosmetic. In other words, at the third step, the powder-attached rod-shaped cosmetic 13 may be cut in a direction crossing the major axis of the powder-attached rod-shaped cosmetic 13 at non-right angle, or might be cut parallel to the major axis of the powder-attached rod-shaped cosmetic 13.

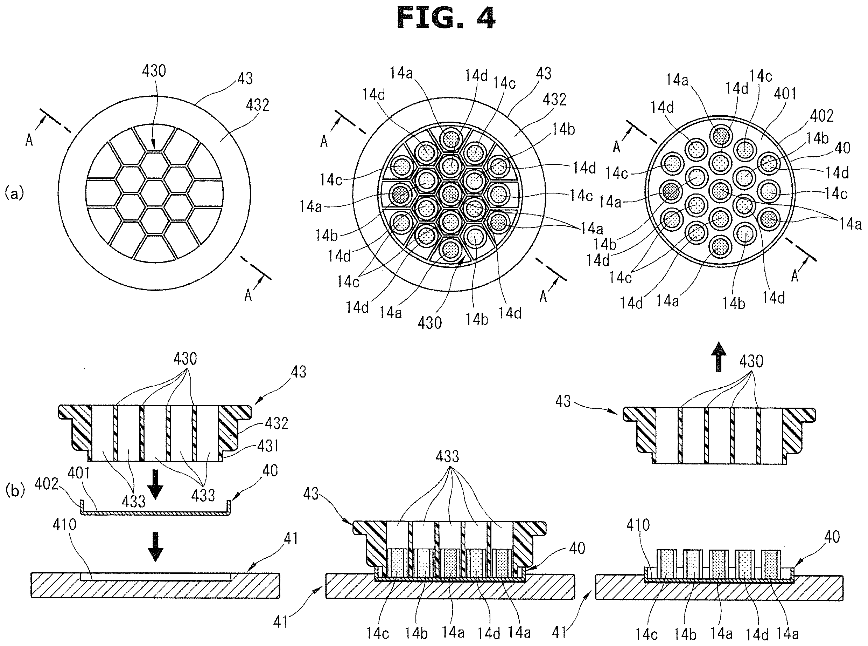

Figs. 4(a) and 4(b) are schematic diagrams illustrating a fourth step of the producing method of the multi-color cosmetic according to the first embodiment. Fig. 4 (a) is a plan view, and Fig. 4(b) is a sectional view taken along an A-A line of Fig. 4(a).

As illustrated in Figs. 4 (a) and4 (b), at the fourth step (a cosmetic arranging step) of the producing method of the multi-color cosmetic, first, the housing tray 40 that is open upward in the vertical direction is set in a circular recess 410 provided on an upper surface in the middle of a substantially thick plate-shaped holder member 41, and a jig 43 provided with a partition 430 is inserted to an inside of the set housing tray 40 from above in the vertical direction. The jig 43 is formed so that its diameter decreases stepwise from one end side of the jig 43 toward the other end side. The jig 43 then has, as integral portions, an insertion portion 431 that is formed at a top end side of the jig 43 into a minimum diameter shape and that can be inserted to the inside of the housing tray 40 and a base portion 432 that is formed at a base end side of the jig 43 into a large diameter shape and that is seated on an upper end portion of the peripheral wall 402 of the housing tray 40.

Subsequently, the unit cosmetics 14a, 14b, 14c and 14d of each color are inserted into the housing tray 40 through a plurality of insertion holes 433 formed by the partition 430 of the jig 43, and these unit cosmetics 14a, 14b, 14c and 14d are positioned and arranged at predetermined positions. After completing the positioning arrangement of the unit cosmetics 14a, 14b, 14c and 14d of each color, the jig 43 is removed from the housing tray 40. It is noted that the present embodiment exemplifies an example in which the arrangement operation for arranging the unit cosmetics 14a, 14b, 14c and 14d of each color is manually done by a worker (a person) using the jig 43 having the partition 430.

In other words, at this fourth step, it is also possible to automatically arrange the unit cosmetics 14a, 14b, 14c and 14d of each color by robot etc. That is, arrangement of the unit cosmetics 14a, 14b, 14c and 14d of each color is previously programmed, then, based on the program, the robot could position the unit cosmetics 14a, 14b, 14c and 14d of each color at predetermined positions. It is noted that when automatically performing the positioning arrangement of the unit cosmetics 14a, 14b, 14c and 14d of each color using the robot etc., the jig 43 having the partition 430 used for the positioning arrangement of the unit cosmetics 14a, 14b, 14c and 14d of each color is unnecessary, and can be omitted.

In addition, at the fourth step, after positioning and arranging the unit cosmetics 14a, 14b, 14c and 14d of each color, it may be possible to photograph arrangement of the unit cosmetics 14a, 14b, 14c and 14d of each color by, for instance, a camera (not shown) and to judge, based on this captured image, whether the arrangement of the unit cosmetics 14a, 14b, 14c and 14d of each color is good or bad. With this judging manner, accuracy of the positioning arrangement of the unit cosmetics 14a, 14b, 14c and 14d of each color is ensured, thereby improving a yield of the multi-color cosmetic 1.

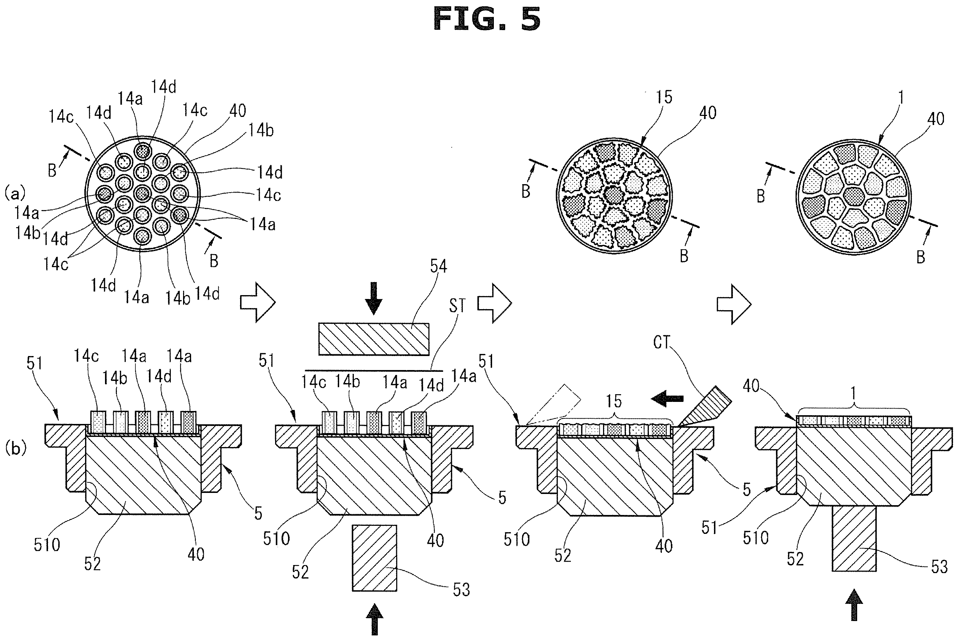

Figs. 5(a) and 5(b) are schematic diagrams illustrating a fifth step and subsequent steps of the producing method of the multi-color cosmetic according to the first embodiment of the present invention. Fig. 5(a) is a plan view, and Fig. 5(b) is a sectional view taken along a B-B line of Fig. 5(a).

As illustrated in Figs. 5(a) and 5(b), at the fifth step (a press molding step) of the producing method of the multi-color cosmetic, first, the housing tray 40 on which the unit cosmetics 14a, 14b, 14c and 14d of each color are positioned and arranged at the fourth step is set in a press machine 5. This press machine 5 has a substantially tubular fixed mold 51 having a mold housing portion 510 formed so as to penetrate a middle portion of the fixedmold51 along the vertical direction, amovable mold 52 slidably housed in the mold housing portion 510 of the fixed mold 51, a ram cylinder 53 disposed below the movable mold 52 in the vertical direction and pressing the movable mold 52 upward in the vertical direction, and a press pad 54 disposed above the movable mold 52 in the vertical direction so as to face the movable mold 52 and pressing each upper end surface of the unit cosmetics 14a, 14b, 14c and 14d of each color with the press pad 54 being in contact with each upper end surface of the unit cosmetics 14a, 14b, 14c and 14d.

Subsequently, when the housing tray 40 is set in the press machine 5, the press pad 54 goes down and comes into contact with the upper end surfaces of the unit cosmetics 14a, 14b, 14c and 14d of each color, and after that, the ram cylinder 53 moves upward, then the movable mold 52 is pressed upward in the vertical direction, i.e. toward the press pad 54. As a result, the housing tray 40 goes upward together with the movable mold 52, and the whole of the unit cosmetics 14a, 14b, 14c and 14d of each color positioned and arranged on this housing tray 40 is sandwiched between the press pad 54 and the movable mold 52. The unit cosmetics 14a, 14b, 14c and 14d of each color are pressed or crushed by this pressing action, then the unit cosmetics 14a, 14b, 14c and 14d of each color spread laterally, and further the unit cosmetics 14a, 14b, 14c and 14d of each color press each other, then the unit cosmetics 14a, 14b, 14c and 14d of each color come into absolute contact with each other and are integral with each other. Aunit cosmetic assembly 15 in which the unit cosmetics 14a, 14b, 14c and 14d of each color are integral with each other is thus formed.

Here, at the fifth step, when pressing the unit cosmetics 14 against the press pad 54, it is preferable to sandwich a paper sheet member ST between the unit cosmetics 14 and the press pad 54. By sandwiching the paper sheet member ST between the unit cosmetics 14 and the press pad 54, there is no risk that each of the unit cosmetics 14 will stick to the press pad 54 upon pressing. As a result, an upper surface of the unit cosmetic assembly 15 after the press can be smoothly formed.

After completing the fifth step, at a sixth step (a surface peeling step) of the producing method of the multi-color cosmetic, by moving a cutter CT in a horizontal direction so as to peel an upper end surface of the unit cosmetic assembly 15 having been pressed or crushed by the pressing action, an upper end portion of the unit cosmetic assembly 15 is cut thin. As a result, a cross section of the unit cosmetic assembly 15 is exposed, and the multi-color cosmetic 1 having a smooth surface is formed. Afterwards, an air cylinder 55 rises, and the movable mold 52 is pressed upward in the vertical direction, then the multi-color cosmetic 1 is discharged together with the housing tray 40, thereby completing the production of the multi-color cosmetic 1.

(Operation and Effect of the Present Embodiment) The conventional producing method of the multi-color cosmetic does not take into consideration at all that in order to obtain the powder-attached rod-shaped cosmetic, attachment (or adhesion) work of the powdered cosmetic cosmetic, such as rotating (rolling) the rod-shaped cosmetic on the powdered cosmetic filling the container, is automated. Therefore, there is room for improvement in that regard.

In contrast to this, since the producing method of the multi-color cosmetic 1 according to the present embodiment provides the following operation and effect, the problem of the conventional producing method of the multi-color cosmetic can be solved.

That is, according to the producing method of the multi-color cosmetic 1 according to the present embodiment, at the powder attaching step as the second step, since the rod-shaped cosmetic 11 rolls on the powdered cosmetic 12 laid on the conveyor 31 by and according to movement of the conveyor 31 with the pressing force applied to the rod-shaped cosmetic 11 from the side facing the conveyor 31, the attachment work of the powdered cosmetic 12 to the rod-shaped cosmetic 11 can be automated. With this, productivity and quality of the multi-color cosmetic 1 can be improved.

Further, since the powdered cosmetic 12 is attached to the outer peripheral surface of the rod-shaped cosmetic 11 by rolling the rod-shaped cosmetic 11 on the powdered cosmetic 12, it is possible to thoroughly or evenly attach the powdered cosmetic 12 to the outer peripheral side of the rod-shaped cosmetic 11, thereby contributing to improvement in quality of the multi-color cosmetic 1.

Furthermore, in the present embodiment, at the second step, the pressing jig 32 is set to the inclined state so that the pressing jig 32 comes into contact with the rod-shaped cosmetic 11 at the end portion of the pressing jig 32 on the opposite side to the travelling direction of the conveyor 31, whereas the pressing jig 32 does not come into contact with the rod-shaped cosmetic 11 (the powder-attached rod-shaped cosmetic 13) at the end portion of the pressing jig 32 in the travelling direction of the conveyor 31. Therefore, the powder-attached rod-shaped cosmetic 13 to which the powdered cosmetic 12 has been attached at the end portion of the pressing jig 32 on the opposite side to the travelling direction of the conveyor 31 can be smoothly carried (conveyed) in the travelling direction of the conveyor 31. This suppresses decrease in an amount of the powdered cosmetic 12 which is caused by contact of the powder-attached rod-shaped cosmetic 13 with the pressing jig 32 after attaching the powdered cosmetic 12, thereby obtaining the powder-attached rod-shaped cosmetic 13 to which the powdered cosmetic 12 is sufficiently attached.

### (Modified Example)

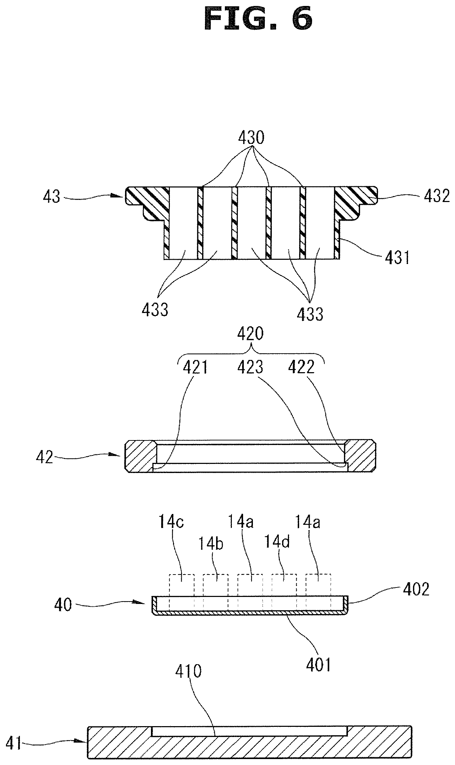

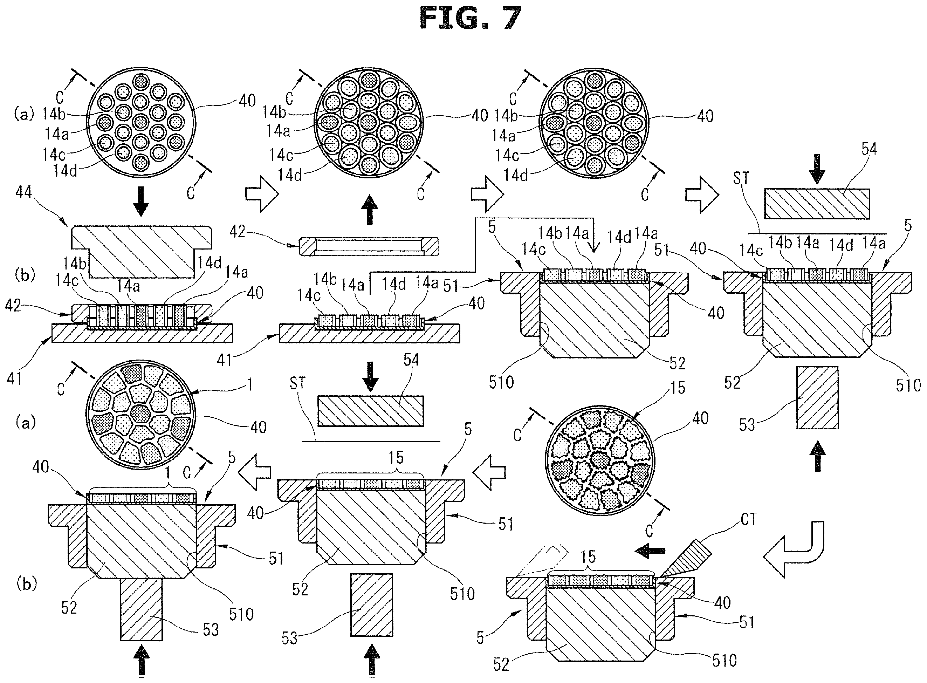

Fig. 6 is a modified example of the first embodiment of the producing method of the multi -color cosmetic according to the present invention, and illustrates a sectional view showing a structure of a jig used at the fourth step of the producing method of the multi-color cosmetic. Figs. 7(a) and 7(b) are schematic diagrams illustrating the fifth step and the subsequent steps of the producing method of the multi-color cosmetic according to the modified example. Fig. 7(a) is a plan view, and Fig. 7(b) is a sectional view taken along a C-C line of Fig. 7(a).

As illustrated in Fig. 6, in the present modified example, at the fourth step of the producing method of the multi-color cosmetic, the housing tray 40 is set in a recess 410 formed on an upper surface in the middle of a holder member 41, and a substantially annular side ring 42 having, at an inside thereof, a penetration hole 420 that can receive the housing tray 40 is disposed at an outer circumferential side of the set housing tray 40. Next, a jig 43 having a partition 430 is inserted from above in the vertical direction through the penetration hole 420 of this side ring 42, and the unit cosmetics 14a, 14b, 14c and 14d of each color are positioned and arranged on the housing tray 40 through a plurality of insertion holes 433 formed by the partition 430 of the jig 43. After completing the positioning arrangement of the unit cosmetics 14a, 14b, 14c and 14d of each color, the jig 43 is removed from the housing tray 40.

Here, the penetration hole 420 of the side ring 42 is formed into a stepped diameter shape, and has a large diameter portion 421 provided at an end portion, facing the housing tray 40, of the penetration hole 420 and protecting an outer circumferential side of the peripheral wall 402 of the housing tray 40, a small diameter portion 422 formed into a step-reduced diameter shape with respect to the large diameter portion 421 and extending an inside diameter of the peripheral wall 402 of the housing tray 40, and a step portion 423 formed between the large diameter portion 421 and the small diameter portion 422 and being able to be seated on an upper end portion of the peripheral wall 402 of the housing tray 40.

Further, the jig 43 is formed so that its diameter decreases stepwise from one end side of the jig 43 toward the other end side. The jig 43 then has, as integral portions, an insertion portion 431 that is formed at a top end side of the jig 43 into a minimum diameter shape and that can be inserted to the inside of the housing tray 40 and a base portion 432 that is formed at a base end side of the jig 43 into a large diameter shape and that is seated on an upper end surface of the side ring 42.

Subsequently, as illustrated in Figs. 7(a) and 7(b), in the present modified example, at the fifth step, a preliminary press pad 44 goes down with respect to the housing tray 40 mounted in the recess 410 of the holder member 41 and held by the side ring 42, and a top end portion of the preliminary press pad 44 is inserted into the penetration hole 420 of the side ring 42, then as a so-called preliminary press, the unit cosmetics 14a, 14b, 14c and 14d of each color positioned and arranged on the housing tray 40 are pressed relatively shallowly. More specifically, the unit cosmetics 14a, 14b, 14c and 14d of each color having been positioned and arranged are pressed to such an extent that the unit cosmetics 14a, 14b, 14c and 14d of each color are in contact with each other, then the preliminary press is completed. After completing this preliminary press, the side ring 42 is removed, and the housing tray 40 is separated from the holder member 41 and is set in the press machine 5. Here, since the configuration or structure of the press machine 5 is the same as that of the first embodiment, its detailed description is omitted here.

Next, when the housing tray 40 is set in the press machine 5, the press pad 54 goes down and comes into contact with the upper end surfaces of the unit cosmetics 14a, 14b, 14c and 14d of each color, and after that, the ram cylinder 53 moves upward, then the movable mold 52 is pressed toward the press pad 54. With this pressing, the whole of the unit cosmetics 14a, 14b, 14c and 14d of each color having undergone the preliminary press is sandwiched between the press pad 54 and the movable mold 52 and pressed or crushed by the pressing action, and the unit cosmetics 14a, 14b, 14c and 14d of each color further spread laterally, then the unit cosmetics 14a, 14b, 14c and 14d of each color come into absolute contact with each other and are integral with each other. As a result, the unit cosmetic assembly 15 in which the unit cosmetics 14a, 14b, 14c and 14d of each color are integral with each other is formed. Here, at the fifth step, in the same manner as the first embodiment, it is preferable to sandwich the paper sheet member ST between the unit cosmetics 14a, 14b, 14c and 14d and the press pad 54.

Subsequently, after completing the fifth step, at the sixth step of the producing method of themulti-color cosmetic, an upper end portion of the unit cosmetic assembly 15 formed at the fifth step is cut thin by the cutter CT. With this cutting, a cross section of the unit cosmetic assembly 15 is exposed. Next, after completing the sixth step, at a seventh step (a finish-pressing step) of the producing method of the multi-color cosmetic, the cross section of the unit cosmetic assembly 15, which is exposed at the sixth step, undergoes a finishing-press by being slightly pressed by the press pad 54. With this finishing-press, the multi-color cosmetic 1 having a smoother surface than the cross section exposed by the cutterCTis formed. Afterwards, the air cylinder 55 rises, and the movable mold 52 is pressed upward in the vertical direction, then the multi-color cosmetic 1 is discharged together with the housing tray 40, thereby completing the production of the multi-color cosmetic 1.

As described above, in the present modified example, at the fifth step, before performing the press molding, a preliminary molding by which the unit cosmetics 14a, 14b, 14c and 14d of each color are pressed relatively shallowly is performed. By performing the two-step press molding of the unit cosmetics 14a, 14b, 14c and 14d of each color in this way, the unit cosmetics 14a, 14b, 14c and 14d of each color are pressed stepwise. Therefore, the unit cosmetics 14a, 14b, 14c and 14d of each color blend in better with each other, and the unit cosmetics 14a, 14b, 14c and 14d of each color can come into absolute contact with each other well. As a result, quality of the multi-color cosmetic 1 can be further improved.

In addition, in the present modified example, after the sixth step, by pressing the exposed cross section of the unit cosmetic assembly 15 by the finishing-press, the surface of the multi-color cosmetic 1 is adjusted. Surface roughness of the multi-color cosmetic 1 can therefore be smoothed more, thereby improving a sense of use of the multi-color cosmetic 1.

### [Second Embodiment]

### (Description of Method of Producing Multi-color cosmetic)

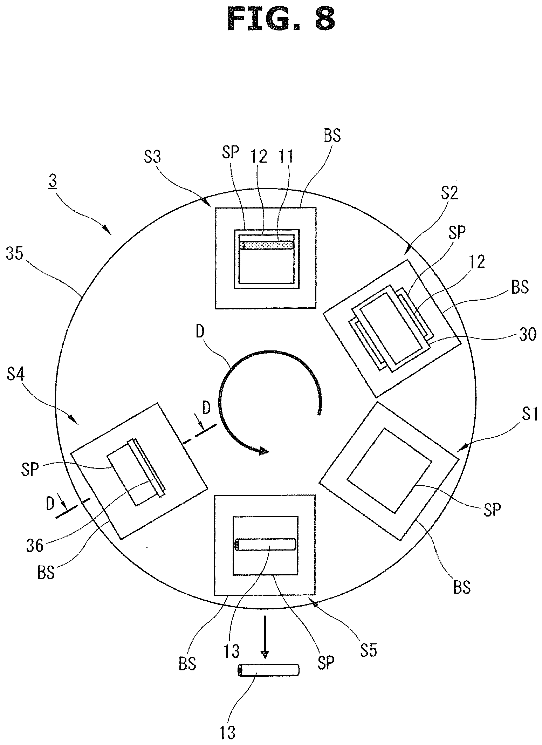

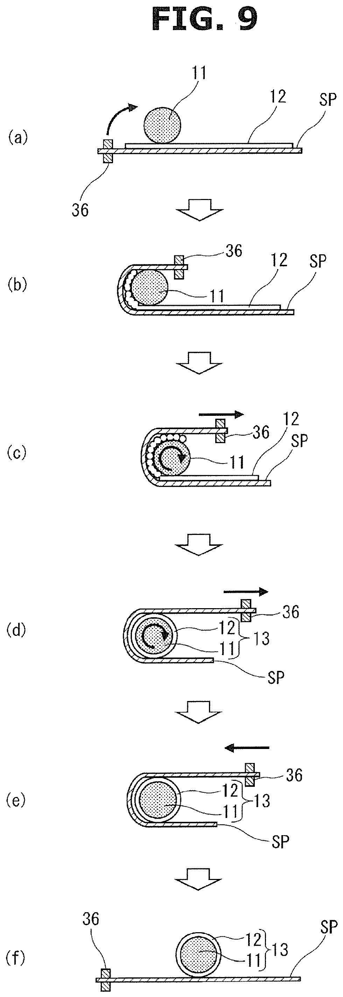

Fig. 8 and Figs. 9(a) to 9(f) show a second embodiment of the producing method of the multi-color cosmetic according to the present invention. In the present embodiment, the second step of the producing method of the multi-color cosmetic according to the first embodiment is changed. Since each step other than this change is the same as that of the first embodiment, in the present embodiment, only the change will be described, and descriptions of the same steps as those of the first embodiment are omitted here.

Fig. 8 shows the second embodiment of the producing method of the multi-color cosmetic according to the present invention, and illustrates a plan view of a powder attaching device showing another example of the second step of the producing method of the multi-color cosmetic. Figs. 9(a) to 9(f) are sectional views taken along a D-D line of Fig. 8, and schematically illustrate an attachment (or adhesion) operation of the powdered cosmetic 12 to the rod-shaped cosmetic 11.

As illustrated in Fig. 8, the second step of the producing method of the multi-color cosmetic according to the second embodiment has a sheet arranging step S1 at which a sheet SP is arranged, a powdered cosmetic filling step S2 at which the powdered cosmetic 12 is laid (spread) on the sheet SP, a rod-shaped cosmetic arranging step S3 at which the rod-shaped cosmetic 11 is arranged on the powdered cosmetic 12 laid on the sheet SP, a rod-shaped cosmetic rolling step S4 at which the rod-shaped cosmetic 11 is made to roll on the powdered cosmetic 12 by rolling up the sheet SP then the powder-attached rod-shaped cosmetic 13 is obtained, and a powder-attached cosmetic discharging step S5 at which the powder-attached rod-shaped cosmetic 13 obtained is discharged. That is, a powder attaching device 3 shown in Fig. 8 has a substantially circular turntable 35 that can turn, and on this turntable 35, the sheet arranging step S1, the powdered cosmetic filling step S2, the rod-shaped cosmetic arranging step S3, the rod-shaped cosmetic rolling step S4 and the powder-attached cosmetic discharging step S5 are arranged in a turning direction (in a counterclockwise direction) of the turntable 35 indicated by an arrow D.

At the sheet arranging step S1, the substantially rectangular sheet SP is arranged on a base BS placed at a first angular position (a position of a 4 o'clock direction in Fig. 8) on the turntable 35. Here, the sheet SP is formed of material to which cosmetic (the rod-shaped cosmetic 11 and the powdered cosmetic 12) is not easily attached, and is configured so as not to interfere with formation of the powder-attached rod-shaped cosmetic 13.

At the powdered cosmetic filling step S2, the powdered cosmetic 12 is laid (spread) on a surface of the sheet SP arranged on the base BS positioned at a second angular position (a position of a 2 o'clock direction in Fig. 8) that is turned from the sheet arranging step S1 in the counterclockwise direction. At this time, the powdered cosmetic 12 is stored in the powder container 30 provided at the second angular position above the turntable 35 so as to be able to move along a radial direction of the turntable 35. The powdered cosmetic 12 is then supplied to the surface of the sheet SP so as to be sprinkled onto the surface of the sheet SP from a lower portion of the powder container 30 moving along the radial direction of the turntable 35.

At the rod-shaped cosmetic arranging step S3, the rod-shaped cosmetic 11 is laid and arranged on the powdered cosmetic 12 laid on the surface of the sheet SP on the base BS positioned at a third angular position (a position of a 12 o'clock direction in Fig. 8) that is turned from the powdered cosmetic filling step S2 in the counterclockwise direction. At this time, the rod-shaped cosmetic 11 is placed in a direction orthogonal to a direction in which the sheet SP is rolled up at the following step, i.e. the rod-shaped cosmetic 11 is placed along a width direction of the sheet SP. It is noted that as long as the rod-shaped cosmetic 11 is laid and placed in a direction orthogonal to a direction in which the sheet SP is rolled up then the rod-shaped cosmetic 11 rolls at the following step, a direction in which the rod-shaped cosmetic 11 is laid can be arbitrarily changed according to specifications etc. of the powder attaching device 3.

At the rod-shaped cosmetic rolling step S4, the sheet SP arranged on the base BS positioned at a fourth angular position (a position of an 8 o'clock direction in Fig. 8) that is turned from the rod-shaped cosmetic arranging step S3 in the counterclockwise direction is rolled up from a radially inner side toward a radially outer side of the turntable 35. More specifically, as illustrated in Fig. 8 and Figs. 9(a) to 9(f), one end portion (an end portion at the radially inner side of the turntable 35 in the present embodiment), in a direction orthogonal to a longitudinal direction of the rod-shaped cosmetic 11, of the sheet SP is sandwiched and grasped by a rolling machine 36 (see Fig. 9(a)). Then, the one end portion of the sheet SP is folded back to the other end side (see Fig. 9(b)), and the end portion of the sheet SP is pulled toward the other end side (see Figs. 9(c) and 9(d)), then the rod-shaped cosmetic 11 is made to roll on the powdered cosmetic 12, thereby forming the powder-attached rod-shaped cosmetic 13. After that, the sheet SP is opened so that the grasped end portion of the sheet SP is returned to the one end side (see Fig. 9(e)), and the powder-attached rod-shaped cosmetic 13 is placed at a middle portion of the flat sheet SP (see Fig. 9(f)).

At the powder-attached cosmetic discharging step S5, the powder-attached rod-shaped cosmetic 13 on (above) the base BS positioned at a fifth angular position (a position of a 6 o'clock direction in Fig. 8) that is turned from the rod-shaped cosmetic rolling step S4 in the counterclockwise direction is discharged. At this time, it is preferable to discharge the powder-attached rod-shaped cosmetic 13 through the sheet SP arranged on the base BS. That is, by discharging the powder-attached rod-shaped cosmetic 13 through the sheet SP, the sheet SP can also be discharged together with the powder-attached rod-shaped cosmetic 13, which eliminates the need to remove the sheet SP separately. It is therefore possible to immediately arrange a new sheet SP on the base BS at the next step, thereby improving working efficiency in production.

(Operation and Effect of the Present Embodiment) As described above, according to the present embodiment, at the second step as the powder attaching step, after laying and arranging the rod-shaped cosmetic 11 on the powdered cosmetic 12 laid on the sheet SP, by folding back the one end portion of the sheet SP to the other end side and rolling the rod-shaped cosmetic 11 on the powdered cosmetic 12, the attachment work of the powdered cosmetic 12 to the rod-shaped cosmetic 11 can be automated. With this, productivity and quality of the multi-color cosmetic 1 can be improved.

Further, in the present embodiment, by rolling the rod-shaped cosmetic 11 on the powdered cosmetic 12, the powder-attached rod-shaped cosmetic 13 is obtained. Therefore, by controlling the number of rotations (the number of times of rolling) of the rod-shaped cosmetic 11, an amount of the powdered cosmetic 12 attached to the rod-shaped cosmetic 11 can be controlled relatively easily.

Furthermore, in the present embodiment, by folding back the sheet SP, the rod-shaped cosmetic 11 is made to roll on the powdered cosmetic 12, then the powder-attached rod-shaped cosmetic 13 is obtained. Therefore, a shape of the rod-shaped cosmetic 11 could be, for instance, a square columnar shape other than a cylindrical columnar shape, then a degree of freedom of the shape of the rod-shaped cosmetic 11 can be increased.

### (Modified Example)

(1) The above embodiment exemplifies, as an example of the present invention, an example in which one color (single layer) powdered cosmetic 12 has been laid on the sheet SP, then the rod-shaped cosmetic 11 is made to roll on this one color (single layer) powdered cosmetic 12 by rolling up the sheet SP. However, the present invention is not limited to this example. In other words, as another example, a plurality of colors of the powdered cosmetics 12 are previously laid so as to be layered on the sheet SP, and the rod-shaped cosmetic 11 is made to roll once on the plurality of layered powdered cosmetics 12 by rolling up the sheet SP, then a multi-color powder-attached rod-shaped cosmetic 13 to which the plurality of layered powdered cosmetics 12 are attached is produced. Further, it is also possible to produce a multi-color powder-attached rod-shaped cosmetic 13 to which a plurality of spirally layered powdered cosmetics 12 are attached by rolling the rod-shaped cosmetic 11 multiple times on the plurality of layered powdered cosmetics 12.
(2) The above embodiment exemplifies, as an example of the present invention, an example in which the single (one color) rod-shaped cosmetic 11 is made to roll on the powdered cosmetic 12 laid on the sheet SP by rolling up the sheet SP. However, the present invention is not limited to this example. In other words, as another example, a plurality of colors of the rod-shaped cosmetics 11 are arranged on the powdered cosmetic 12 laid on the sheet SP, and these rod-shaped cosmetics 11 are made to roll and be tightened together by rolling up the sheet SP, then a powder-attached multi-color rod-shaped cosmetic in which the plurality of colors of the rod-shaped cosmetics 11 are wrapped like a so-called thick roll is produced.

### [Third Embodiment]

### (Description of Method of Producing Multi-color cosmetic)

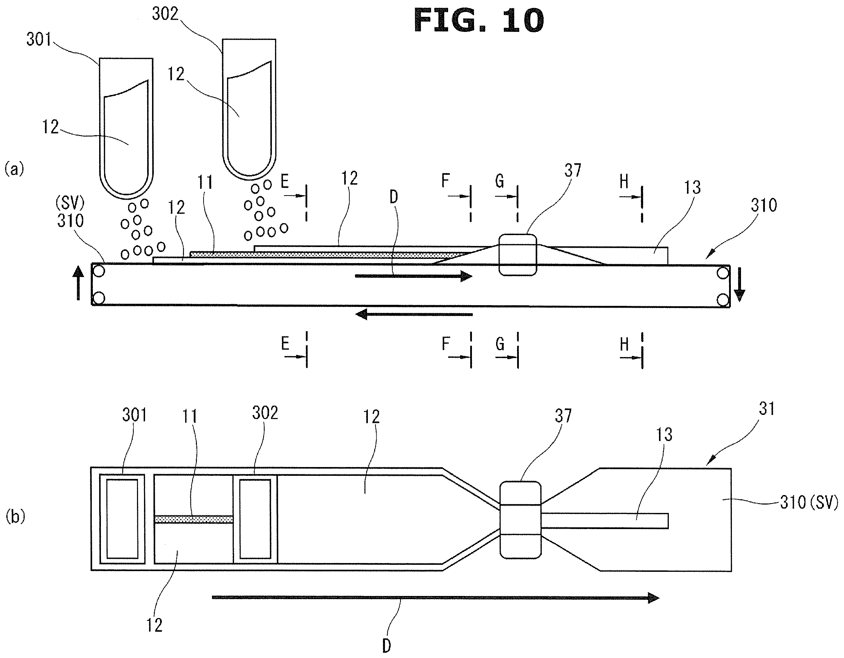

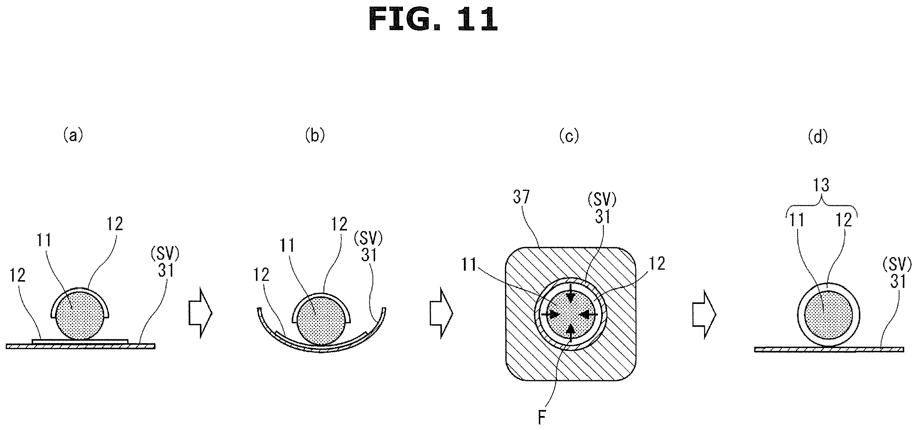

Figs. 10(a) and 10(b) and Figs. 11(a) to 11(d) show a third embodiment of the producing method of the multi-color cosmetic according to the present invention. In the present embodiment, the second step of the producing method of the multi-color cosmetic according to the first embodiment is changed. Since each step other than this change is the same as that of the first embodiment, in the present embodiment, only the change will be described, and descriptions of the same steps as those of the first embodiment are omitted here.

Figs. 10(a) and 10(b) show the third embodiment of the producing method of the multi-color cosmetic according to the present invention. Fig. 10(a) is a side view of the second step, and Fig. 10(b) is a plan view of the second step. Fig. 11(a) is a sectional view taken along an E-E line of Fig. 10(a). Fig. 11(b) is a sectional view taken along an F-F line of Fig. 10(a). Fig. 11(c) is a sectional view taken along a G-G line of Fig. 10(a). Fig. 11(d) is a sectional view taken along an H-H line of Fig. 10(a).

As illustrated particularly in Figs. 10(a) and 10(b), a powder attaching device 3 according to the present embodiment has a conveyor 31 rotating in a direction of an arrow D indicated in Figs. 10(a) and 10(b) along a longitudinal direction of the conveyor 31, a first powder container 301 provided at an end portion on an upstream side of the conveyor 31 and storing therein a powdered cosmetic 12, a constricting part 37 provided on a downstream side of the conveyor 31 and forming a powder-attached rod-shaped cosmetic 13, and a second powder container 302 provided between the first powder container 301 and the constricting part 37 in a travelling direction of the conveyor 31 so as to be movable and storing therein a powdered cosmetic 12.

That is, at the second step of the producing method of the multi-color cosmetic according to the present embodiment, on the upstream side of the conveyor 31 formed by a strip-shaped (or a belt-shaped) sheet SV rotating in the direction of the arrow D indicated in Figs. 10(a) and 10(b) along the longitudinal direction of the conveyor 31, the powdered cosmetic 12 is laid (spread) on an upper surface 310 of the conveyor 31. At this time, the powdered cosmetic 12 is stored in the first powder container 301 disposed at the end portion on an opposite side to the travelling direction of the conveyor 31 (the upstream end portion of the conveyor 31), and is supplied onto the conveyor 31 from a lower portion of the first powder container 301.

Further, after arranging the rod-shaped cosmetic 11 along the travelling direction of the conveyor 31 on the powdered cosmetic 12 laid on the upper surface 310 of the conveyor 31, the powdered cosmetic 12 is further sprinkled onto this rod-shaped cosmetic 11 from above, then the rod-shaped cosmetic 11 is buried in the powdered cosmetic 12. At this time, the powdered cosmetic 12 sprinkled from above the rod-shaped cosmetic 11 is stored in the second powder container 302 provided movably toward the opposite side to the travelling direction of the conveyor 31 above the conveyor 31, and is supplied onto the conveyor 31 from a lower portion of the second powder container 302 moving toward the opposite side to the travelling direction of the conveyor 31.

Subsequently, both sides in a width direction of the conveyor 31 are rolled up (or rounded) at and by the constricting part 37 provided on the downstream side of the conveyor 31 so that both side portions in the width direction of the conveyor 31 overlap each other, then the powdered cosmetic 12 is attached to an outer peripheral side of the rod-shaped cosmetic 11. The constricting part 37 has a tunnel shape having a substantially circular penetration hole 370 along the travelling direction of the conveyor 31, and is configured so that the both sides in the width direction of the conveyor 31 are rolled up as the conveyor 31 passes through the penetration hole 370.

More specifically, as illustrated in Figs. 11(a) and 11(b), the both sides in the width direction of the conveyor 31 mounting thereon the rod-shaped cosmetic 11 buried in the powdered cosmetic 12 are gradually rolled up (or rounded) toward the constricting part 37. Further, as illustrated in Fig. 11(c), the both side portions in the width direction of the conveyor 31 overlap each other at and by the constricting part 37, then the outer peripheral side of the rod-shaped cosmetic 11 is completely wrapped or surrounded with the conveyor 31 having been rolled up (or rounded) so that a cross section of the conveyor 31 substantially becomes circular in shape. As a result, the powdered cosmetic 12 laid on the upper surface of the conveyor 31 is substantially evenly attached to the outer peripheral side of the rod-shaped cosmetic 11, then the powder-attached rod-shaped cosmetic 13 is formed.

It is noted that when surrounding the rod-shaped cosmetic 11 and the powdered cosmetic 12 with the conveyor 31 by rolling up (rounding) the conveyor 31 at and by the constricting part 37, it is preferable to apply an appropriate pressing force F (see Fig. 11(c)) to the powdered cosmetic 12 surrounding the rod-shaped cosmetic 11. By the pressing force Facting on the powdered cosmetic 12, the powdered cosmetic 12 attached to the outer peripheral side of the rod-shaped cosmetic 11 is appropriately compressed and compacted. As a result, falling-off of the powdered cosmetic 12 is suppressed, thereby firming (stabilizing) a shape of the powder-attached rod-shaped cosmetic 13.

When passing through the constricting part 37, a round state of the both sides in the width direction of the conveyor 31 is gradually released, and as illustrated in Fig. 11(d), at the downstream end portion of the conveyor 31, the powder-attached rod-shaped cosmetic 13 remains and is placed at a middle portion of the conveyor 31. That is, most of the powdered cosmetic 12 laid on the upper surface of the conveyor 31 on the upstream side of the conveyor 31 is attached to the outer peripheral side of the rod-shaped cosmetic 11 on a downstream side of the constricting part 37, then only the powder-attached rod-shaped cosmetic 13 remains and is placed on the upper surface of the conveyor 31 at the downstream end portion of the conveyor 31. Here, the powder-attached rod-shaped cosmetic 13 placed at the downstream end portion of the conveyor 31 is discharged by, for instance, a discharge conveyor (not shown) disposed adjacently to the downstream end portion of the conveyor 31.

(Operation and Effect of the Present Embodiment) As described above, according to the present embodiment, at the second step as the powder attaching step, after arranging the rod-shaped cosmetic 11 along the travelling direction of the conveyor 31 on the conveyor 31 (the sheet SV) on which the powdered cosmetic 12 is laid then burying the rod-shaped cosmetic 11 in the powdered cosmetic 12 by further sprinkling the powdered cosmetic 12 onto the rod-shaped cosmetic 11 from above, by rolling up (or rounding) the both sides in the width direction of the conveyor 31 at and by the constricting part 37 provided on the downstream side of the conveyor 31 so that both side portions in the width direction of the conveyor 31 overlap each other, the attachment work of the powdered cosmetic 12 to the rod-shaped cosmetic 11 can be automated. With this, productivity and quality of the multi-color cosmetic 1 can be improved.

Further, in the present embodiment, the rod-shaped cosmetic 11 is arranged along the travelling direction of the conveyor 31. Therefore, the powdered cosmetic 12 can be attached to a relatively long rod-shaped cosmetic 11. As a result, a relatively long powder-attached rod-shaped cosmetic 13 can be obtained. It is thus possible to cut the relatively long powder-attached rod-shaped cosmetic 13 into a required unit length, thereby improving working efficiency in production of the unit cosmetic 14.

### [Fourth Embodiment]

### (Description of Method of Producing Multi-color cosmetic)

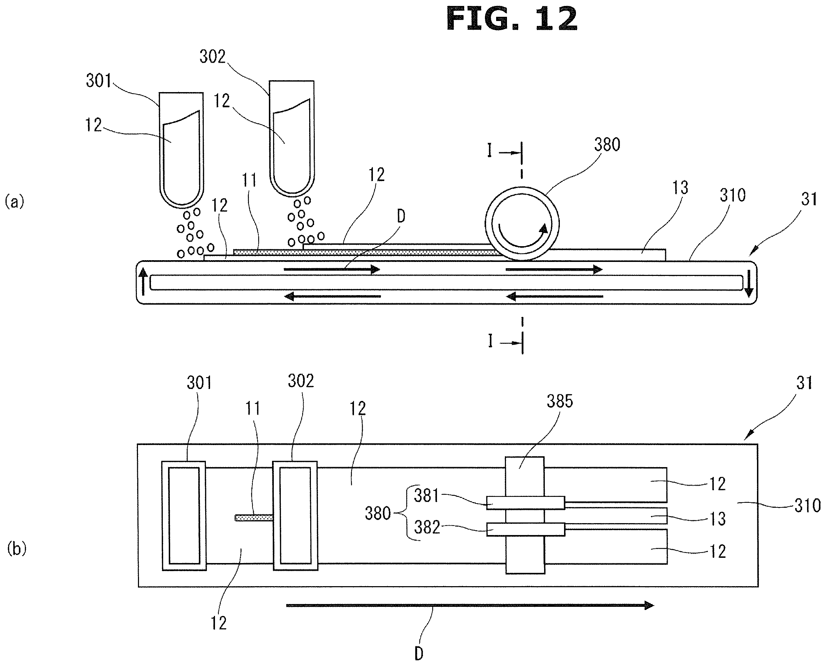

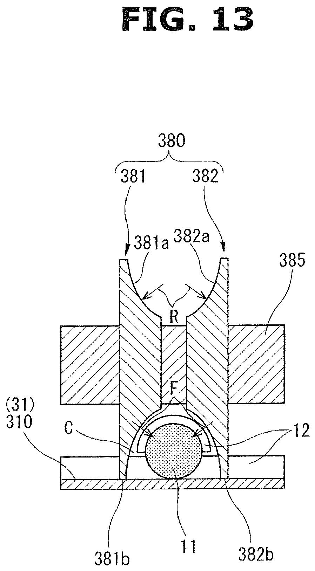

Figs. 12(a) and 12(b) and Fig. 13 show a fourth embodiment of the producing method of the multi-color cosmetic according to the present invention. In the present embodiment, the second step of the producing method of the multi-color cosmetic according to the first embodiment is changed. Since each step other than this change is the same as that of the first embodiment, in the present embodiment, only the change will be described, and descriptions of the same steps as those of the first embodiment are omitted here.

Figs. 12(a) and 12(b) show the fourth embodiment of the producing method of the multi-color cosmetic according to the present invention. Fig. 12(a) is a side view of the second step, and Fig. 12(b) is a plan view of the second step. Fig. 13 is a sectional view taken along an I-I line of Fig. 12(a).

As illustrated particularly in Figs. 12(a) and 12(b), a powder attaching device 3 according to the present embodiment has a conveyor 31 rotating in a direction of an arrow D indicated in Figs. 12(a) and 12(b) along a longitudinal direction of the conveyor 31, a first powder container 301 provided at an end portion on an upstream side of the conveyor 31 and storing therein a powdered cosmetic 12, a constricting part 38 provided on a downstream side of the conveyor 31 and forming a powder-attached rod-shaped cosmetic 13, and a second powder container 302 provided between the first powder container 301 and the constricting part 38 in a travelling direction of the conveyor 31 so as to be movable and storing therein a powdered cosmetic 12.

That is, at the second step of the producing method of the multi-color cosmetic according to the present embodiment, on the upstream side of the conveyor 31 rotating in the direction of the arrow D indicated in Figs. 12(a) and 12(b) along the longitudinal direction of the conveyor 31, the powdered cosmetic 12 is laid (spread) on an upper surface 310 of the conveyor 31. At this time, the powdered cosmetic 12 is stored in the first powder container 301 disposed at the end portion on an opposite side to the travelling direction of the conveyor 31 (the upstream end portion of the conveyor 31), and is supplied onto the conveyor 31 from a lower portion of the first powder container 301.

Further, after arranging the rod-shaped cosmetic 11 along the travelling direction of the conveyor 31 on the powdered cosmetic 12 laid on the upper surface 310 of the conveyor 31, the powdered cosmetic 12 is further sprinkled onto this rod-shaped cosmetic 11 from above, then the rod-shaped cosmetic 11 is buried in the powdered cosmetic 12. At this time, the powdered cosmetic 12 sprinkled from above the rod-shaped cosmetic 11 is stored in the second powder container 302 provided movably toward the opposite side to the travelling direction of the conveyor 31 above the conveyor 31, and is supplied onto the conveyor 31 from a lower portion of the second powder container 302 moving toward the opposite side to the travelling direction of the conveyor 31.

Subsequently, the powdered cosmetic 12 attached to an outer peripheral side of the rod-shaped cosmetic 11 is scraped off at and by the constrictingpart 38 provided on the downstream side of the conveyor 31, then the powder-attached rod-shaped cosmetic 13 is formed. The constricting part 38 is configured by a roller 380 that rotates in a direction opposite to the conveyor 31.

In the present embodiment, the roller 380 is configured so that a pair of first and second rollers 381 and 382 having substantially arc-shaped concave arc surfaces 381a and 382a that are concave outwards in the width direction of the conveyor 31 respectively are arranged so as to face each other in the width direction of the conveyor 31, and are connected and integral with each other by a supporting shaft 385. Here, the concave arc surfaces 381a and 382a are set to a predetermined curvature R corresponding to an outside diameter of the powder-attached rod-shaped cosmetic 13. Thefirstroller 381 and the second roller 382 are arranged so that outer edge portions 381b and 382b of the first and second rollers 381 and 382 are in sliding contact with (or close to) the upper surface 310 of the conveyor 31, and the first and second rollers 381 and 382 are driven and rotate through the supporting shaft 385. The first and second rollers 381 and 382 form, in cooperation with each other, a substantially semicircular gap (a tunnel) C penetrating along the travelling direction (see the arrow D in Figs. 12(a) and 12(b)) of the conveyor 31 between these first and second rollers 381 and 382 and the conveyor 31.

More specifically, as illustrated in Figs. 12 (a) and 12 (b) and Fig. 13, the first roller 381 and the second roller 382 connected through the supporting shaft 385 integrally (synchronously) rotate, and the rod-shaped cosmetic 11 buried in the powdered cosmetic 12 carried (conveyed) by the conveyor 31 passes through the gap C between the first roller 381 and the second roller 382, then the powder-attached rod-shaped cosmetic 13 having a substantially semicircular cross section corresponding to the gap C is formed.

At this time, it is preferable to apply a pressing force (an urging force) F to the roller 380 formed by the first roller 381 and the second roller 382 toward the conveyor 31 and to pressurize the powder-attached rod-shaped cosmetic 13 from its outer peripheral side. By pressurizing the powder-attached rod-shaped cosmetic 13 from its outer peripheral side by applying the pressing force F to the roller 380 in this manner, the powdered cosmetic 12 attached to the outer peripheral side of the rod-shaped cosmetic 11 is compressed and compacted. As a result, falling-off of the powdered cosmetic 12 is suppressed, thereby firming (stabilizing) a shape of the powder-attached rod-shaped cosmetic 13.

When passing through the constricting part 38, at a downstream side of the constricting part 38, the powder-attached rod-shaped cosmetic 13 shaped into a shape of the gap C remains and is placed at a middle portion of the conveyor 31 with the powdered cosmetic 12 accumulating at both sides in the width direction of the conveyor 31. That is, at the downstream side of the constricting part 38, an excess powdered cosmetic 12 attached to the outer peripheral side of the rod-shaped cosmetic 11 is scraped off, and the powder-attached rod-shaped cosmetic 13 with an appropriate amount of the powdered cosmetic 12 attached to the outer peripheral side of the rod-shaped cosmetic 11 remains and is placed. Here, the powder-attached rod-shaped cosmetic 13 formed at the downstream side of the constricting part 38 is discharged by, for instance, a discharge conveyor (not shown) disposed adjacently to the downstream end portion of the conveyor 31.

(Operation and Effect of the Present Embodiment) As described above, according to the present embodiment, at the second step as the powder attaching step, after arranging the rod-shaped cosmetic 11 along the travelling direction of the conveyor 31 on the conveyor 31 on which the powdered cosmetic 12 is laid then burying the rod-shaped cosmetic 11 in the powdered cosmetic 12 by further sprinkling the powdered cosmetic 12 onto the rod-shaped cosmetic 11 from above, by making the rod-shaped cosmetic 11 buried in the powdered cosmetic 12 pass through the gap C between the roller 380 (the first roller 381 and the second roller 382) and the conveyor 31 at the constricting part 38 provided on the downstream side of the conveyor 31, the attachment work of the powdered cosmetic 12 to the rod-shaped cosmetic 11 can be automated. With this, productivity and quality of the multi-color cosmetic 1 can be improved.

Further, in the present embodiment, only by making the rod-shaped cosmetic 11 buried in the powdered cosmetic 12 pass through the gap C of the roller 380 (between the first roller 381 and the second roller 382), automation of the attachment work of the powdered cosmetic 12 to the rod-shaped cosmetic 11 can be relatively easily realized.

Furthermore, in the present embodiment, the rod-shaped cosmetic 11 is arranged along the travelling direction of the conveyor 31. Therefore, the powdered cosmetic 12 can be attached to a relatively long rod-shaped cosmetic 11. As a result, a relatively long powder-attached rod-shaped cosmetic 13 can be obtained. It is thus possible to cut the relatively long powder-attached rod-shaped cosmetic 13 into a required unit length, thereby improving working efficiency in production of the unit cosmetic 14.

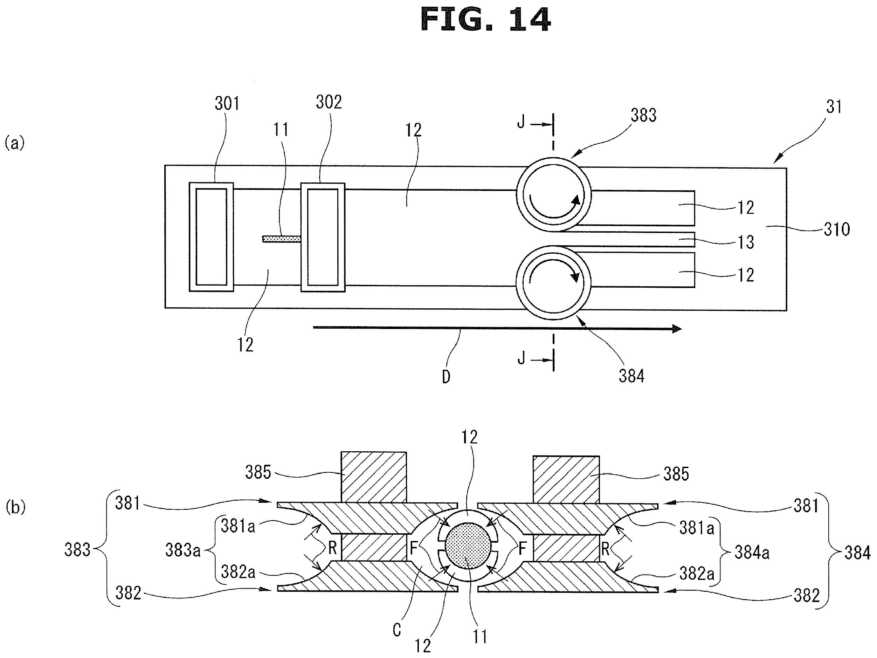

Moreover, in the present embodiment, in order to obtain the powder-attached rod-shaped cosmetic 13, it is only necessary to make the rod-shaped cosmetic 11 buried in the powdered cosmetic 12 pass through the gap C between the pair of the first roller 381 and the second roller 382 arranged so as to face the conveyor 31, which does not require floating the rod-shaped cosmetic 11 from the conveyor 31 (see Fig. 14(b)) when passing through the constricting part 38 as described in, for instance, the following modified example. With this, the attachment work of the powdered cosmetic 12 to the rod-shaped cosmetic 11 can be relatively easily realized.

### (Modified Example)

Figs. 14(a) and 14(b) are a modified example of the producing method of the multi-color cosmetic according to the fourth embodiment of the present invention. Fig. 14(a) is a plan view of the second step, and Fig. 14(b) is a sectional view taken along a J-J line of Fig. 14 (a) .

As illustrated in Figs. 14(a) and 14(b), in the present modified example, the constricting part 38 according to the fourth embodiment is configured by a pair of left roller 383 and right roller 384 arranged so as to face each other in the width direction of the conveyor 31. Each of the left and right rollers 383 and 384 is configured by the pair of first roller 381 and second roller 382 connected through the supporting shaft 385 disclosed in the fourth embodiment. These left and right rollers 383 and 384 have substantially semicircular concave arc surfaces 383a and 384a that are concave outwards in the width direction of the conveyor 31 respectively, and form a substantially circular gap (a tunnel) C penetrating along the travelling direction (see the arrow D in Fig. 14(a)) of the conveyor 31 between both the rollers 383 and 384. Here, each of the concave arc surfaces 383a and 384a is formed by combining the concave arc surface 381a of the first roller 381 and the concave arc surface 382a of the second roller 382, and has a predetermined curvature R corresponding to an outside diameter of the powder-attached rod-shaped cosmetic 13.

More specifically, as illustrated in Figs. 14 (a) and 14(b), the left roller 383 and the right roller 384 rotate in opposite directions along the travelling direction of the conveyor 31, and the rod-shaped cosmetic 11 buried in the powdered cosmetic 12 carried (conveyed) by the conveyor 31 passes through the gap C between the left roller 383 and the right roller 384, then the powder-attached rod-shaped cosmetic 13 having a substantially circular cross section corresponding to the gap C is formed.

At this time, it is preferable to apply a pressing force (an urging force) F to the left roller 383 and the right roller 384 against each other and to pressurize the powder-attached rod-shaped cosmetic 13 from its outer peripheral side. By pressurizing the powder-attached rod-shaped cosmetic 13 from its outer peripheral side by applying the pressing force F to the left roller 383 and the right roller 384 in this manner, the powdered cosmetic 12 attached to the outer peripheral side of the rod-shaped cosmetic 11 is compressed and compacted. As a result, falling-off of the powdered cosmetic 12 is suppressed, thereby firming (stabilizing) a shape of the powder-attached rod-shaped cosmetic 13.

As described above, according to the present modified example, in order to obtain the powder-attached rod-shaped cosmetic 13, by making the rod-shaped cosmetic 11 buried in the powdered cosmetic 12 pass through the substantially circular gap C formed by the pair of rollers 383 and 384 arranged so as to face each other in the width direction of the conveyor 31, the powder-attached rod-shaped cosmetic 13 having the circular cross section can be directly obtained. In other words, there is no need to modify an outside shape of the powder-attached rod-shaped cosmetic 13 having the semicircular cross section, which is obtained by, for instance, the fourth embodiment, into a circular cross section, and the powder-attached rod-shaped cosmetic 13 having the circular cross section can be efficiently formed.

The present invention is not limited to the configurations or structures exemplified in the above embodiments and examples. As long as the operation (working) and the effect of the present invention described above can be obtained, the present invention can be freely changed according to specification, cost etc. of objects to which the present invention is applied.

In particular, coloring of the multi-color cosmetic 1 is not limited to four colors exemplified in the above embodiments and examples, and if the coloring is a plurality of colors of two colors or more, the coloring can be arbitrarily changed according to pattern or design formed in the multi-color cosmetic 1.

Further, as an example of the second step (the powder attaching step) of the producing method of the multi-color cosmetic according to the present invention, the example in which the single layer (one layer) powdered cosmetic 12 is attached to the outer peripheral side of the rod-shaped cosmetic 11 is illustrated. However, the present invention is not limited to this example, i.e. the example in which the single layer (one layer) powdered cosmetic 12 is attached to the outer peripheral side of the rod-shaped cosmetic 11. In other words, after forming the powder-attached rod-shaped cosmetic 13, the powdered cosmetic 12 to be used is changed (for instance, color etc. are changed), and by further attaching the powdered cosmetic 12 to an outer peripheral side of the powder-attached rod-shaped cosmetic 13 again by the attaching method described in the above embodiments, a multi-color powder-attached rod-shaped cosmetic to which concentrically multi-layered powdered cosmetics 12 are attached could be formed.

Furthermore, regarding the cutting operation of the powder-attached rod-shaped cosmetic 13 carried out at the third step, it is not limited to the example in which the powder-attached rod-shaped cosmetic 13 is cut for each of the unit cosmetics 14a, 14b, 14c and 14d of each color as described in the above embodiments. For instance, the powder-attached rod-shaped cosmetics 13 are bundled together and accommodated in a tubular case, and by cutting an assembly of these bundled powder-attached rod-shaped cosmetics 13, plate-shape multi-color cosmetic bulks could be formed. In this case, since the plate-shape multi-color cosmetic bulk obtained can be placed on the housing tray 40 and immediately set in and pressed by the press machine 5, productivity of the multi-color cosmetic 1 can be improved.

In addition, with regard to the method of positioning and arranging the plurality of powder-attached unit cosmetics 14 at the fourth step, the jig 43 having the partition 430 exemplified in the above embodiments can be used, and this positioning arrangement could be automated by robot etc.

Additionally, in the first embodiment and the second embodiment of the present invention, the example in which the rod-shaped cosmetic 11 is made to roll on the conveyor 31 or the sheet SP on which the single-color powdered cosmetic 12 is laid is illustrated. However, the present invention is not limited to this example. In other words, as another example of the first embodiment and the second embodiment, a plurality of colors of the powdered cosmetics 12 are laid in a striped pattern on the conveyor 31 or the sheet SP, and by rolling the rod-shaped cosmetic 11 on the powdered cosmetics 12 laid in the striped pattern, a powder-attached rod-shaped cosmetic 13 in which the plurality of powdered cosmetics 12 having a so-called horizontal stripe pattern (a so-called border pattern) are attached to the outer peripheral side of the rod-shaped cosmetic 11 could be formed.

Further, in the another example of the first embodiment and the second embodiment, when laying the plurality of colors of the powdered cosmetics 12 in the striped pattern, in addition to the horizontal stripe pattern (the border pattern) formed by laying the plurality of colors of the powdered cosmetics 12 in a direction perpendicular to a rolling direction of the rod-shaped cosmetic 11, for instance, the plurality of colors of the powdered cosmetics 12 are laid in a direction obliquely to the rolling direction of the rod-shaped cosmetic 11, and by rolling the rod-shaped cosmetic 11 on the powdered cosmetics 12, a powder-attached rod-shaped cosmetic 13 in which the powdered cosmetics 12 having an oblique stripe pattern are attached to the outer peripheral side of the rod-shaped cosmetic 11 maybe formed. In other words, by laying the plurality of colors of the powdered cosmetics 12 in an arbitrarily pattern on the conveyor 31 or the sheet SP and rolling the rod-shaped cosmetic 11 on the powdered cosmetics 12, the powdered cosmetics 12 having an arbitrarily pattern can be attached to the outer peripheral side of the rod-shaped cosmetic 11.

### EXPLANATION OF REFERENCE

1···multi-color cosmetic, 11···rod-shaped cosmetic, 12···powdered cosmetic, 13···powder-attached rod-shaped cosmetic, 14···unit cosmetic, 14a···unit cosmetic, 14b···unit cosmetic, 14c···unit cosmetic, 14d···unit cosmetic, 15···unit cosmetic assembly, 2···extrusion mold machine, 21···screw, 3···powder attaching device, 30···powder container, 31···conveyor, 32···pressing jig, 33···inclined plate, 34···discharge tray, 35···turntable, 36···rolling machine, 37···constricting part, 38···constricting part, 380···roller, 381···first roller, 382···second roller, 383···first roller, 384···second roller, 41···holder member, 42···side ring, 43···jig, 430···partition, 5···press machine, 51···fixed mold, 52···movable mold, 53···ram cylinder, 54···press pad, 55···air cylinder, CT···cutter, C···gap, SP···sheet, SV···sheet

## Claims

1. A method of producing a multi-color cosmetic comprising:
a powder attaching step of attaching a powdered cosmetic (12) to each outer peripheral side of rod-shaped cosmetics (11) forming multiple colors, wherein the rod-shaped cosmetics (11) are each formed by shaping respective cosmetics forming multiple colors of two colors or more into a rod-shape; and
a press molding step of compressing and compacting an assembly of a plurality of parallel-arranged unit cosmetics (14) forming multiple colors, wherein the unit cosmetics (14) are obtained by cutting each of powder-attached rod-shaped cosmetics (13) forming multiple colors, which are formed by attaching the powdered cosmetic (12) to the outer peripheral sides of the rod-shaped cosmetics (11) forming multiple colors, in a direction orthogonal to a longitudinal direction of the powder-attached rod-shaped cosmetic (13),
**characterized in that** the powder attaching step includes:
a powdered cosmetic filling step (S2) at which the powdered cosmetic (12) is laid on a sheet (SP);
a rod-shaped cosmetic arranging step (S3) at which the rod-shaped cosmetic (11) is laid and arranged on the powdered cosmetic (12) laid on the sheet (SP); and
a rod-shaped cosmetic rolling step (S4) at which by grasping one end portion, in a direction orthogonal to a longitudinal direction of the rod-shaped cosmetic (11), of the sheet (SP) and folding back the one end portion of the sheet (SP) to the other end side, the rod-shaped cosmetic (11) is made to roll on the powdered cosmetic (12), and the powder-attached rod-shaped cosmetic (13) is formed.

2. A method of producing a multi-color cosmetic comprising:
a powder attaching step of attaching a powdered cosmetic (12) to each outer peripheral side of rod-shaped cosmetics (11) forming multiple colors, wherein the rod-shaped cosmetics (11) are each formed by shaping respective cosmetics forming multiple colors of two colors or more into a rod-shape; and
a press molding step of compressing and compacting an assembly of a plurality of parallel-arranged unit cosmetics (14) forming multiple colors, wherein the unit cosmetics (14) are obtained by cutting each of powder-attached rod-shaped cosmetics (13) forming multiple colors, which are formed by attaching the powdered cosmetic (12) to the outer peripheral sides of the rod-shaped cosmetics (11) forming multiple colors, in a direction orthogonal to a longitudinal direction of the powder-attached rod-shaped cosmetic (13),
**characterized in that** at the powder attaching step,
on an upstream side of a conveyor (31) formed by a strip-shaped sheet (SV) rotating along a longitudinal direction of the conveyor (31), the powdered cosmetic (12) is laid on the conveyor (31),
the rod-shaped cosmetic (11) is arranged along a travelling direction of the conveyor (31) on the powdered cosmetic (12) laid on the conveyor (31),
by further sprinkling the powdered cosmetic (12) onto the rod-shaped cosmetic (11) from above, the rod-shaped cosmetic (11) is buried in the powdered cosmetic (12), and
on a downstream side of the conveyor (31), by rolling up both side portions in a width direction of the conveyor (31) so that the both side portions of the conveyor (31) overlap each other, the powder-attached rod-shaped cosmetic (13) is formed.

3. A method of producing a multi-color cosmetic comprising:
a powder attaching step of attaching a powdered cosmetic (12) to each outer peripheral side of rod-shaped cosmetics (11) forming multiple colors, wherein the rod-shaped cosmetics (11) are each formed by shaping respective cosmetics forming multiple colors of two colors or more into a rod-shape; and
a press molding step of compressing and compacting an assembly of a plurality of parallel-arranged unit cosmetics (14) forming multiple colors, wherein the unit cosmetics (14) are obtained by cutting each of powder-attached rod-shaped cosmetics (13) forming multiple colors, which are
formed by attaching the powdered cosmetic (12) to the outer peripheral sides of the rod-shaped cosmetics (11) forming multiple colors, in a direction orthogonal to a longitudinal direction of the powder-attached rod-shaped cosmetic (13),
**characterized in that** at the powder attaching step,
on an upstream side of a conveyor (31) formed by a strip-shaped sheet (SV) rotating along a longitudinal direction of the conveyor (31), the powdered cosmetic (12) is laid on the conveyor (31),
the rod-shaped cosmetic (11) is arranged along a travelling direction of the conveyor (31) on the powdered cosmetic (12) laid on the conveyor (31),
by further sprinkling the powdered cosmetic (12) onto the rod-shaped cosmetic (11) from above, the rod-shaped cosmetic (11) is buried in the powdered cosmetic (12), and
on a downstream side of the conveyor (31), by making the rod-shaped cosmetic (11) buried in the powdered cosmetic (12) pass through an arc-shaped or a circular gap (C) between a pair of rollers ( 381,382 ; 383,384) that are arranged as a pair so as to face each other in a width direction of the conveyor (31) and that form the arc-shaped or circular gap (C) between the pair of rollers (381,382; 383,384), the powder-attached rod-shaped cosmetic (13) is formed.

4. The method of producing the multi-color cosmetic as claimed in claim 3, wherein
the pair of rollers (381,382) are configured so that concave arc surfaces (381a, 382a) that are concave outwards in the width direction of the conveyor (31) are arranged so as to face each other in the width direction of the conveyor (31), and so that the arc-shaped gap (C) is formed between the pair of rollers (381,382), also a semicircular gap (C) is formed between the pair of rollers (381,382) and the conveyor (31).

5. The method of producing the multi-color cosmetic as claimed in claim 3, wherein
the pair of rollers (383,384) are configured so that semicircular concave arc surfaces (383a, 384a) that are concave outwards in the width direction of the conveyor (31) are arranged so as to face each other in the width direction of the conveyor (31), and so that the circular gap (C) is formed between the pair of rollers (383,384).

## Patentansprüche

1. Verfahren zum Herstellen eines mehrfarbigen Kosmetikums, umfassend:
einen Pulveraufbringungsschritt zum Aufbringen eines pulverförmigen Kosmetikums (12) an den Außenumfangsseiten von stabförmigen Kosmetika (11) für mehrere Farben, wobei die stabförmigen Kosmetika (11) jeweils durch das Formen von entsprechenden Kosmetika für mehrere Farben, d.h. zwei oder mehr Farben, zu einer Stabform ausgebildet werden, und
einen Pressformschritt zum Komprimieren und Verdichten einer Anordnung einer Vielzahl von parallel angeordneten Einheitskosmetika (14) für mehrere Farben, wobei die Einheitskosmetika (14) durch das Schneiden von stabförmigen Kosmetika (13) für mehrere Farben mit einem daran aufgebrachtem Pulver, die durch das Aufbringen des pulverförmigen Kosmetikums (12) an den Außenumfangsseiten der stabförmigen Kosmetika (11) für mehrere Farben ausgebildet werden, in einer Richtung orthogonal zu einer Längsrichtung des stabförmigen Kosmetikums (13) mit dem daran aufgebrachten Pulver erhalten werden,
**dadurch gekennzeichnet, dass** der Pulveraufbringungsschritt umfasst:
einen Pulverförmiges-Kosmetikum-Füllschritt (S2), in dem das pulverförmige Kosmetikum (12) auf ein Blatt (SP) gelegt wird,
einen Stabförmiges-Kosmetikum-Anordnungsschritt (S3), in dem das stabförmige Kosmetikum (11) auf das auf das Blatt (SP) gelegte pulverförmige Kosmetikum (12) gelegt und auf diesem angeordnet wird, und
einen Stabförmiges-Kosmetikum-Rollschritt (S4), in dem durch das Greifen eines Endteils des Blatts (SP) in einer Richtung orthogonal zu der Längsrichtung des stabförmigen Kosmetikums (11) und das Zurückfalten des Endteils des Blatts (SP) zu der anderen Endseite das stabförmige Kosmetikum (11) auf das pulverförmige Kosmetikum (12) gerollt wird und das stabförmige Kosmetikum (13) mit dem daran aufgebrachten Pulver ausgebildet wird.

2. Verfahren zum Herstellen eines mehrfarbigen Kosmetikums, umfassend:
einen Pulveraufbringungsschritt zum Aufbringen eines pulverförmigen Kosmetikums (12) an den Außenumfangsseiten von stabförmigen Kosmetika (11) für mehrere Farben, wobei die stabförmigen Kosmetika (11) jeweils durch das Formen von entsprechenden Kosmetika für mehrere Farben, d.h. zwei oder mehr Farben, zu einer Stabform ausgebildet werden, und
einen Pressformschritt zum Komprimieren und Verdichten einer Anordnung einer Vielzahl von parallel angeordneten Einheitskosmetika (14) für mehrere Farben, wobei die Einheitskosmetika (14) durch das Schneiden von stabförmigen Kosmetika (13) für mehrere Farben mit einem daran aufgebrachtem Pulver, die durch das Aufbringen des pulverförmigen Kosmetikums (12) an den Außenumfangsseiten der stabförmigen Kosmetika (11) für mehrere Farben ausgebildet werden, in einer Richtung orthogonal zu einer Längsrichtung des stabförmigen Kosmetikums (13) mit dem daran aufgebrachten Pulver erhalten werden,
**dadurch gekennzeichnet, dass** in dem Pulveraufbringungsschritt:
auf einer stromaufwärts gelegenen Seite einer Fördereinrichtung (31), die durch ein sich entlang der Längsrichtung der Fördereinrichtung (31) drehendes streifenförmiges Blatt (SV) gebildet wird, das pulverförmige Kosmetikum (12) auf die Fördereinrichtung (31) gelegt wird,
das stabförmige Kosmetikum (11) entlang einer Bewegungsrichtung der Fördereinrichtung (31) auf dem auf die Fördereinrichtung (31) gelegten pulverförmigen Kosmetikum (12) angeordnet wird,
indem weiterhin das pulverförmige Kosmetikum (12) von oben auf das stabförmige Kosmetikum (11) gestreut wird, das stabförmige Kosmetikum (11) in dem pulverförmigen Kosmetikum (12) vergraben wird, und
auf einer stromabwärts gelegenen Seite der Fördereinrichtung (31) durch das Hochrollen beider Seitenteile in einer Breitenrichtung der Fördereinrichtung (31), sodass die beiden Seitenteile der Fördereinrichtung (31) miteinander überlappen, das pulverförmige Kosmetikum (13) mit dem daran aufgebrachten Pulver ausgebildet wird.

3. Verfahren zum Herstellen eines mehrfarbigen Kosmetikums, umfassend:
einen Pulveraufbringungsschritt zum Aufbringen eines pulverförmigen Kosmetikums (12) an den Außenumfangsseiten von stabförmigen Kosmetika (11) für mehrere Farben, wobei die stabförmigen Kosmetika (11) jeweils durch das Formen von entsprechenden Kosmetika für mehrere Farben, d.h. zwei oder mehr Farben, zu einer Stabform ausgebildet werden, und
einen Pressformschritt zum Komprimieren und Verdichten einer Anordnung einer Vielzahl von parallel angeordneten Einheitskosmetika (14) für mehrere Farben, wobei die Einheitskosmetika (14) durch das Schneiden von stabförmigen Kosmetika (13) für mehrere Farben mit einem daran aufgebrachtem Pulver, die durch das Aufbringen des pulverförmigen Kosmetikums (12) an den Außenumfangsseiten der stabförmigen Kosmetika (11) für mehrere Farben ausgebildet werden, in einer Richtung orthogonal zu einer Längsrichtung des stabförmigen Kosmetikums (13) mit dem daran aufgebrachten Pulver erhalten werden,
**dadurch gekennzeichnet, dass** in dem Pulveraufbringungsschritt:
auf einer stromaufwärts gelegenen Seite einer Fördereinrichtung (31), die durch ein sich entlang der Längsrichtung der Fördereinrichtung (31) drehendes streifenförmiges Blatt (SV) gebildet wird, das pulverförmige Kosmetikum (12) auf die Fördereinrichtung (31) gelegt wird,
das stabförmige Kosmetikum (11) entlang einer Bewegungsrichtung der Fördereinrichtung (31) auf dem auf die Fördereinrichtung (31) gelegten pulverförmigen Kosmetikum (12) angeordnet wird,
indem weiterhin das pulverförmige Kosmetikum (12) von oben auf das stabförmige Kosmetikum (11) gestreut wird, das stabförmige Kosmetikum (11) in dem pulverförmigen Kosmetikum (12) vergraben wird, und
auf einer stromabwärts gelegenen Seite der Fördereinrichtung (31) durch das Hindurchführen des in dem pulverförmigen Kosmetikum (12) vergrabenen stabförmigen Kosmetikums (11) durch einen bogenförmigen oder kreisförmigen Zwischenraum (C) zwischen einem Paar von Rollen (381, 382; 383, 384), die als ein Paar derart angeordnet sind, dass sie in einer Breitenrichtung der Fördereinrichtung (31) einander zugewandt sind und den bogenförmigen oder kreisförmigen Zwischenraum (C) zwischen dem Paar von Rollen (381, 382; 383, 384) bilden, das stabförmige Kosmetikum (13) mit dem daran aufgebrachten Pulver ausgebildet wird.

4. Verfahren zum Herstellen eines mehrfarbigen Kosmetikums nach Anspruch 3, wobei:
das Paar von Rollen (381, 382) derart konfiguriert sind, dass konkave Bogenflächen (381a, 382a), die konkav nach außen in der Breitenrichtung der Fördereinrichtung (31) sind, derart angeordnet sind, dass sie einander in der Breitenrichtung der Fördereinrichtung (31) zugewandt sind und der bogenförmige Zwischenraum (C) zwischen dem Paar von Rollen (381, 382) gebildet wird und auch ein halbkreisförmiger Zwischenraum (C) zwischen dem Paar von Rollen (381, 382) und der Fördereinrichtung (31) gebildet wird.

5. Verfahren zum Herstellen eines mehrfarbigen Kosmetikums nach Anspruch 3, wobei:
das Paar von Rollen (383, 384) derart konfiguriert sind, dass halbkreisförmige konkave Bogenflächen (383a, 384a), die konkav nach außen in der Breitenrichtung der Fördereinrichtung (31) sind, derart angeordnet sind, dass sie einander in der Breitenrichtung der Fördereinrichtung (31) zugewandt sind und der kreisförmige Zwischenraum (C) zwischen dem Paar von Rollen (383, 384) gebildet wird.

## Revendications

1. Procédé de production d'un produit cosmétique multicolore, comprenant :
une étape de fixation de poudre consistant à fixer un produit cosmétique en poudre (12) sur chaque côté périphérique extérieur de produits cosmétiques en forme de tige (11) formant de multiples couleurs, dans lequel les produits cosmétiques en forme de tige (11) sont chacun formés par mise en forme de produits cosmétiques respectifs formant de multiples couleurs, constituées de deux couleurs ou plus, en une forme de tige ; et
une étape de moulage par compression consistant à comprimer et à compacter un ensemble constitué d'une pluralité de produits cosmétiques unitaires (14) disposés parallèlement formant de multiples couleurs, dans lequel les produits cosmétiques unitaires (14) sont obtenus en découpant chacun de produits cosmétiques en forme de tige auxquels est fixée de la poudre (13) formant de multiples couleurs, qui sont formés par fixation du produit cosmétique en poudre (12) sur les côtés périphériques extérieurs des produits cosmétiques en forme de tige (11) formant de multiples couleurs, dans une direction orthogonale à une direction longitudinale du produit cosmétique en forme de tige auquel est fixée de la poudre (13),
**caractérisé en ce que** l'étape de fixation de poudre comporte :
une étape (S2) de remplissage de produit cosmétique en poudre dans laquelle le produit cosmétique en poudre (12) est déposé sur une feuille (SP) ;
une étape (S3) d'agencement de produit cosmétique en forme de tige dans laquelle le produit cosmétique en forme de tige (11) est déposé et agencé sur le produit cosmétique en poudre (12) déposé sur la feuille (SP) ; et
une étape (S4) de roulage de produit cosmétique en forme de tige dans laquelle, en saisissant une partie d'extrémité, dans une direction orthogonale à une direction longitudinale du produit cosmétique en forme de tige (11), de la feuille (SP) et en rabattant ladite partie d'extrémité de la feuille (SP) sur l'autre côté d'extrémité, le produit cosmétique en forme de tige (11) est amené à rouler sur le produit cosmétique en poudre (12), et le produit cosmétique en forme de tige auquel est fixée de la poudre (13) est formé.

2. Procédé de production d'un produit cosmétique multicolore, comprenant :
une étape de fixation de poudre consistant à fixer un produit cosmétique en poudre (12) sur chaque côté périphérique extérieur de produits cosmétiques en forme de tige (11) formant de multiples couleurs, dans lequel les produits cosmétiques en forme de tige (11) sont chacun formés par mise en forme de produits cosmétiques respectifs formant de multiples couleurs, constituées de deux couleurs ou plus, en une forme de tige ; et
une étape de moulage par compression consistant à comprimer et à compacter un ensemble constitué d'une pluralité de produits cosmétiques unitaires (14) disposés parallèlement formant de multiples couleurs, dans lequel les produits cosmétiques unitaires (14) sont obtenus en découpant chacun de produits cosmétiques en forme de tige auxquels est fixée de la poudre (13) formant de multiples couleurs, qui sont formés par fixation du produit cosmétique en poudre (12) sur les côtés périphériques extérieurs des produits cosmétiques en forme de tige (11) formant de multiples couleurs, dans une direction orthogonale à une direction longitudinale du produit cosmétique en forme de tige auquel est fixée de la poudre (13),
**caractérisé en ce que**, lors de l'étape de fixation de poudre,
sur un côté amont d'un convoyeur (31) formé par une feuille en forme de bande (SV) tournant le long d'une direction longitudinale du convoyeur (31), le produit cosmétique en poudre (12) est déposé sur le convoyeur (31),
le produit cosmétique en forme de tige (11) est agencé le long d'une direction de déplacement du convoyeur (31) sur le produit cosmétique en poudre (12) déposé sur le convoyeur (31),
en saupoudrant en outre le produit cosmétique en poudre (12) sur le produit cosmétique en forme de tige (11) par le dessus, le produit cosmétique en forme de tige (11) est incrusté dans le produit cosmétique en poudre (12), et
sur un côté aval du convoyeur (31), en roulant deux portions latérales dans une direction de largeur du convoyeur (31) de sorte que les deux portions latérales du convoyeur (31) se chevauchent l'une l'autre, le produit cosmétique en forme de tige auquel est fixée de la poudre (13) est formé.

3. Procédé de production d'un produit cosmétique multicolore, comprenant :
une étape de fixation de poudre consistant à fixer un produit cosmétique en poudre (12) sur chaque côté périphérique extérieur de produits cosmétiques en forme de tige (11) formant de multiples couleurs, dans lequel les produits cosmétiques en forme de tige (11) sont chacun formés par mise en forme de produits cosmétiques respectifs formant de multiples couleurs, constituées de deux couleurs ou plus, en une forme de tige ; et
une étape de moulage par compression consistant à comprimer et à compacter un ensemble constitué d'une pluralité de produits cosmétiques unitaires (14) disposés parallèlement formant de multiples couleurs, dans lequel les produits cosmétiques unitaires (14) sont obtenus en découpant chacun de produits cosmétiques en forme de tige auxquels est fixée de la poudre (13) formant de multiples couleurs, qui sont formés par fixation du produit cosmétique en poudre (12) sur les côtés périphériques extérieurs des produits cosmétiques en forme de tige (11) formant de multiples couleurs, dans une direction orthogonale à une direction longitudinale du produit cosmétique en forme de tige auquel est fixée de la poudre (13),
**caractérisé en ce que**, lors de l'étape de fixation de poudre,
sur un côté amont d'un convoyeur (31) formé par une feuille en forme de bande (SV) tournant le long d'une direction longitudinale du convoyeur (31), le produit cosmétique en poudre (12) est déposé sur le convoyeur (31),
le produit cosmétique en forme de tige (11) est agencé le long d'une direction de déplacement du convoyeur (31) sur le produit cosmétique en poudre (12) déposé sur le convoyeur (31),
en saupoudrant en outre le produit cosmétique en poudre (12) sur le produit cosmétique en forme de tige (11) par le dessus, le produit cosmétique en forme de tige (11) est incrusté dans le produit cosmétique en poudre (12), et
sur un côté aval du convoyeur, en faisant passer le produit cosmétique en forme de tige (11) incrusté dans le produit cosmétique en poudre (12) à travers un interstice (C) circulaire ou en forme d'arc entre une paire de rouleaux (381, 382 ; 383, 384) qui sont agencés en paire de manière à se faire face dans une direction de largeur du convoyeur (31) et qui forment l'interstice (C) circulaire ou en forme d'arc entre la paire de rouleaux (381, 382 ; 383, 384), le produit cosmétique en forme de tige auquel est fixée de la poudre (13) est formé.

4. Procédé de production du produit cosmétique multicolore selon la revendication 3, dans lequel
la paire de rouleaux (381, 382) est configurée de sorte que des surfaces d'arc concaves (381a, 382a) qui sont concaves vers l'extérieur dans la direction de largeur du convoyeur (31) sont agencées de manière à se faire face dans la direction de largeur du convoyeur (31), et de sorte que l'interstice (C) en forme d'arc est formé entre la paire de rouleaux (381, 382), un interstice semi-circulaire (C) est également formé entre la paire de rouleaux (381, 382) et le convoyeur (31).

5. Procédé de production du produit cosmétique multicolore selon la revendication 3, dans lequel
la paire de rouleaux (383, 384) est configurée de sorte que des surfaces d'arc semi-circulaires concaves (383a, 384a) qui sont concaves vers l'extérieur dans la direction de largeur du convoyeur (31) sont agencées de façon à se faire face dans la direction de largeur du convoyeur (31), et de sorte que l'interstice circulaire (C) est formé entre la paire de rouleaux (383, 384).
